# NEW EUROPEAN PATENT SPECIFICATION

(11) **EP 0 842 208 B2**
(45) Date of publication and mention of the opposition decision: **19.08.2009**
(45) Mention of the grant of the patent: 17.05.2000
(21) Application number: 96928015.5
(22) Date of filing: 26.07.1996
(51) Int. Cl.: C08G 61/10, C07C 25/00, C09K 11/06

(54) **2,7-ARYL-9-SUBSTITUTED FLUORENES AND 9-SUBSTITUTED FLUORENE OLIGOMERS AND POLYMERS**
2,7-ARYL-9-SUBSTITUIERTE FLUORENE UND 9-SUBSTITUIERTE FLUORENOLIGOMERE UND POLYMERE
FLUORENES A SUBSTITUTION 2,7-ARYLE EN POSITION 9, OLIGOMERES ET POLYMERES DE FLUORENES SUBSTITUES EN POSITION 9

(30) Priority: 28.07.1995 US 508942; 28.07.1995 US 508943
(43) Date of publication of application: 20.05.1998
(73) Proprietor: Sumitomo Chemical Company, Limited, Chuo-ku Tokyo (JP)
(72) Inventor: WOO, Edmund, P., Midland, MI 48640 (US); INBASEKARAN, Michael, Midland, MI 48640 (US); SHIANG, William, R., Sanford, MI 48657 (US); ROOF, Gordon, R., Midland, MI 48642 (US)
(74) Representative: Burford, Anthony Frederick
(86) International application number: PCT/US1996/012290
(87) International publication number: WO 1997/005184

(56) References cited:
- EP-A- 0 085 185
- EP-A- 0 348 717
- FR-A- 2 702 870

## Description

This invention relates to novel 2,7-dihalofluoreries which are substituted at the 9-position and methods for the preparation of such 9-substituted 2,7-dihalofluorenes. This invention also relates to 2,7-aryl-9-substituted fluorenes and 9-substituted fluorene oligomers and polymers prepared therefrom. The invention further relates to processes for the preparation of such fluorenes, oligomers and polymers. The invention also relates to films and coatings prepared from such fluorenes, oligomers and polymers and processes for preparing such films and coatings.

Polymers and oligomers of fluorenes substituted by alkyl groups at the 9-carbon position have been reported by Fukuda et al., Japanese Journal of Applied Physics, Vol. 28, pp. L1433-L1435 (1989). Such polymers are disclosed as useful as luminescing materials in the preparation of light-emitting diodes. These polymers were prepared by the Kovacic procedure wherein the appropriate fluorene monomers were treated for several days with a large excess of oxidizing metal salts such as ferric chloride. The structures are represented as poly(fluorene-2,7'-diyls). In a later article, Fukuda discloses that the procedure used resulted in significant crosslinking and mislinking reactions during the polymerization: See Fukuda et al., Journal of Polymer Science, Polymer Chemistry Edition, Vol. 31, pp. 2465-2471 (1993). Brown et al., Journal of Polymer Science, Polymer Chemistry Edition, Vol. 24, pp. 255-267 (1989) disclose the presence of substantial chemical defects under the reaction conditions of the Kovacic procedure, in particular, a significant number of polynuclear structures result. In addition, the oxidative polymerization is not regiospecific and the coupling of fluorenes through other positions, such as the 3,5'- and 3,6'-positions, frequently occurs. In addition, it is possible that branching may occur as a result of attachment of more than two other fluorene molecules to a given fluorene molecule, thereby creating trifunctional materials which could crosslink during the preparation process. The presence of such by-products can resulting low molecular weight oligomers and polymers with low degrees of polymerization. Further, such materials demonstrate a high polydispersity and low glass transition temperatures. Such problems result in poor film formation properties and poor properties in any films prepared in that such films may demonstrate unacceptable mechanical properties and low heat resistance. Furthermore, the oxidative coupling process is very slow.

In one aspect, the invention relates to novel 2,7-dihalofluorenes having substituents in the 9-position. In one embodiment, the fluorene is substituted at the 9-position by two moieties selected from C₁₋₂₀ hydrocarbyl moieties and C₁₋₂₀ hydrocarbyl moieties which contain one or more heteroatoms selected from S, N, O, P and Si. In another embodiment, the fluorene is substituted at the 9-position by a C₅₋₂₀ ring structure, a C₄₋₂₀ ring structure containing one or more heteroatoms selected from S, N and O, a hydrocarbylidene moiety or a hydrocarbylidene moiety which may be further substituted. In the embodiment where the 9-position is substituted with a hydrocarbylidene moiety, the carbon of the hydrocarbylidene moiety connected to the carbon at the 9-position is doubly bonded to such carbon.

In a second aspect, this invention relates to 2,7-fluorenyl oligomers and polymers of the above-described compounds, as well as certain crosslinked derivatives thereof.

In another embodiment, the invention relates to 9-substituted fluorene polymers which are terminated at the terminal 2- and 7'-positions with a halogen wherein the polymers have weight average molecular weights of 10,000 or greater and polydispersities of 3.0 or less.

The polymers and oligomers of the invention do not contain a significant amount of misformed polynuclear structures or bonding through positions other than the 2-and 7'-positions. The fluorene polynuclear rings can further be substituted at the 3-, 4-, 5- or 6-positions with substituents which do not adversely affect the properties of the 2,7-aryl-9-substituted fluorenes or 9-substituted fluorene oligomers and polymers or subsequent processing of such materials for their intended uses.

Another embodiment of the invention involves a process for the preparation of 2,7-aryl-9-substituted fluorenes and 9-substituted fluorene oligomers and polymers. The process comprises contacting one or more 2,7-dihalo-9-substituted fluorenes with a haloaromatic compound or haloaromatic compounds, the aromatic group preferably being further substituted with a reactive group capable of crosslinking or chain extension or a trialkyl-siloxy moiety, in the presence of a catalytic amount of a divalent nickel salt, at least a stoichiometric amount of zinc powder and a trihydrocarbylphosphine in a polar solvent and an optional co-solvent comprising an aromatic hydrocarbon or ether, under conditions such that a 2,7-aryl-9-substituted fluorene or a 9-substituted fluorene oligomer or polymer is prepared. Suitably, the divalent nickel salt is present in an amount of from 0.01 to 20 mole percent based on the amount of haloaromatic compound and 2,7-dihalofluorene, zinc powder is present in an amount of 100 mole percent to 300 mole percent based on the haloaromatic compound and 2,7-dihalofluorene, triarylphosphine is present in an amount of from 10 to 50 mole percent based on the haloaromatic compound and 2,7-dihalofluorene and a compound capable of accelerating the reaction is present in an amount from 100 to 150 mole percent based on the divalent salt.

The 9-substituted fluorene oligomers and polymers terminated at the terminal 2- and 7'-positions with a halogen are prepared by the process described above in the absence of a haloaromatic compound.

In another embodiment, the invention comprises films or coatings comprising 2,7-aryl-9-substituted fluorenes or 9-substituted fluorene oligomers or polymers. Such films may be prepared by applying a composition comprising the 2,7-aryl-9-substituted fluorenes or 9-substituted fluorene oligomers or polymers to a substrate and exposing the applied composition to conditions such that a film is prepared.

Further, the 2,7-aryl-9-substituted fluorenes or 9-substituted fluorene oligomers or polymers may be B-staged, partially crosslinked or chain extended, to prepare a composition which may be used to prepare coatings or films as described hereinbefore.

The 2,7-aryl-9-substituted fluorenes or 9-substituted fluorene oligomers or polymers demonstrate fluorescence, high glass transition temperatures or liquid-crystalline properties and facilitate the preparation of films which have high heat resistance and solvent resistance. The 9-substituted fluorene oligomers and polymers demonstrate low polydispersities. Polymers based on 2,7-aryl-9-substituted fluorenes and 9-substituted fluorene oligomers and polymers which have high molecular weights can be prepared if desired. The 2,7-aryl-9-substituted fluorenes or 9-substituted fluorene oligomers or polymers may be used to prepare films or coatings which may be used in polymeric light-emitting diodes, preferably as the emitting layer. Additionally, such films or coatings may be used as protective layers in electronic devices or as fluorescent coatings in a wide variety of uses.

The 2,7-dihalo-9-disubstituted fluorenes are described by Formula I and the 2,7-dihalo-9-hydrocarbylidenylfluorenes are described by Formula II, 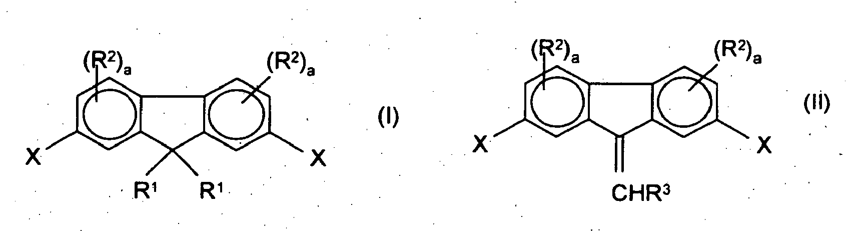 wherein R¹, R², R³, X, and a are as defined below.

In one preferred embodiment, the 2,7-dihalo-9-hydrocarbylidenylfluorene is a 2,7-dihalo-9-benzylidenylfluorene which corresponds to Formula III, 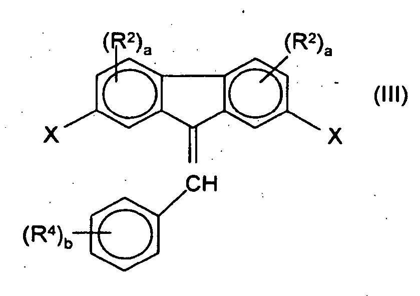 wherein R², R³, R⁴, X, a and b are as defined below.

The compounds of this invention are generally solid, crystalline materials. Generally, the compounds have a melting point in the range of 0°C to 300°C.

Hydrocarbylidene, as used herein, shall mean a hydrocarbyl moiety bound by a double bond to the 9-position of the fluorene ring.

In a preferred embodiment, the 2,7-aryl-9-substituted fluorenes and 9-substituted fluorene oligomers or polymers correspond to Formula IV, 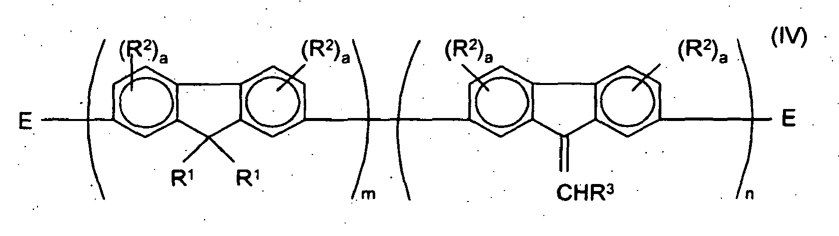 wherein E, R¹, R², R³, X, a, m and n are as defined below and substantially all of the monomer units are connected to end moieties or other monomer units through the 2- and 7-carbon atoms.

E is halogen or an aryl moiety which may optionally be substituted with a reactive group capable of undergoing chain extension or crosslinking, or a trialkylsiloxy group. As used herein, a reactive group capable of undergoing chain extension or crosslinking refers to any group which is capable of reacting with another of the same group or another group so as to form a link to prepare oligomers or polymers. Preferably, such reactive group is a hydroxy, cyanato, glycidyl ether, acrylate ester, methacrylate ester, ethenyl, ethynyl, maleimide, nadimide, trifluorovinyl ether moiety or a benzocyclobutene moiety. E is preferably halogen, aryl or aryl substituted with a reactive group capable of undergoing chain extension or crosslinking or a trialkylsiloxy moiety. E is even more preferably aryl or aryl substituted with a reactive group capable of undergoing chain extension or crosslinking or a trialkylsiloxy. E is most preferably a phenolic, a cyanato-substituted phenyl or a benzocyclobutene moiety.

R¹ is independently in each occurrence C₁₋₂₀ hydrocarbyl; C₁₋₂₀ hydrocarbyl containing one or more heteroatoms selected from S, N, O, P and Si; C₄₋₁₆ hydrocarbyl carbonyloxy, or (C₉₋₁₆ aryl)trialkylsiloxy, or both of R¹ together with the 9-carbon on the fluorene may form a C₅₋₂₀ ring structure or a C₄₋₂₀ ring structure containing one or more heteroatoms selected from S, N, and O. Preferably, R¹ is C₁₋₁₂ alkyl, C₆₋₁₀ aryl or alkyl-substituted aryl, C₄₋₁₆ hydrocarbylcarbonyloxy or (C₉₁₆ aryl)trialkylsiloxy moiety. In the embodiment where the two R¹ form a ring structure with the 9-carbon atom of the fluorene ring, the ring structure formed is preferably, a C₅₋₂₀ straight- or branched-ring structure or a C₄₋₂₀ straight- or branched-chain ring structure containing one or more heteroatoms selected from S, N and O; even more preferably a C₅₋₁₀ aliphatic ring or a C₄₋₁₀ aliphatic ring containing one or more of S or O; and most preferably a C₅₋₁₀ cycloalkyl or C₄₋₁₀ cycloalkyl containing oxygen.

R² is independently in each occurrence a C₁₋₂₀ hydrocarbyl, C₁₋₂₀ hydrocarbyloxy, C₁₋₂₀ thioether, C₁₋₂₀ hydrocarbyloxycarbonyl, C₁₋₂₀ hydrocarbylcarbonyloxy, cyano, thiocyano, C₆₋₁₂ thioaryl, C₁₋₂₀ alkylthio or hydroxy.

R² is preferably C₁₋₁₂ alkyl, C₆₋₁₀ aryl or alkyl-substituted aryl, C₆₋₁₀ aryloxy or alkyl-substituted aryloxy, C₁₋₁₂ alkoxycarbonyl, C₆₋₁₀ aryloxycarbonyl or alkyl-substituted aryloxycarbonyl, C₁₋₁₂ alkoxy, C₁₋₁₂ alkylcarbonyloxy, C₆₋₁₀ arylcarbonyloxy or alkyl-substituted arylcarbonyloxy, cyano, thiocyano, C₆₋₁₂ thioaryl, C₁₋₂₀ alkylthio or hydroxy. Even more preferably, R² is C₁₋₄ alkoxy, phenoxy, C₁₋₄ alkyl, phenyl or cyano.

R³ is independently in each occurrence C₁₋₂₀ hydrocarbyl or C₁₋₂₀ hydrocarbyl substituted with di (C₁₋₂₀ alkyl) amino, C₁₋₂₀ hydrocarbyloxy, C₁₋₂₀ hydrocarbyl or tri (C₁₋₁₀ alkyl) siloxy. R³ is preferably a C₁₋₂₀ straight- or branched-chain aliphatic, a C₁₋₂₀ straight- or branched-chain aliphatic containing one or more cycloaliphatic rings, C₆₋₂₀ aryl or C₇₋₂₀ alkyl-substituted aryl moiety, optionally substituted with a di (C₁₋₂₀ alkyl) amino, C₁₋₂₀ hydrocarbyl, tri(C₁₋₁₀ alkyl) siloxy or C₁₋₂₀ hydrocarbyloxy moiety. R³ is more preferably a C₃₋₁₀ aliphatic, a C₃₋₁₀ aliphatic containing one or more cycloaliphatic moieties, phenyl or phenyl substituted with di (C₁₋₁₂ alkyl) amino; C₁₋₁₀ alkoxy, C₆₋₁₀ aryloxy or alkyl-substituted aryloxy, C₁₋₁₀ alkyl or C₆₋₁₀ aryl or alkyl-substituted aryl or tri (C₁₋₄ alkyl)siloxy. Even more preferably, R³ is phenyl or phenyl, substituted with di (C₁₋₆ alkyl) amino, C₁₋₁₀ alkoxy or C₁₋₁₀ alkyl.

m and n are 0 or non-negative numbers and n+m >1 provided that E is a phenolic, a cyanato-substituted phenyl or a benzocyclobutene moietyor otherwise n+m ≥ 10 and further that m is independently in each occurrence a number of from 1 to 100. n is independently in each occurrence a number of from 0 to 100..

X is halogen, preferably chlorine or bromine; but most preferably bromine.

a is independently in each occurrence 0 or 1.

In a preferred embodiment, the 2,7-aryl-9-substituted fluorenes and 9-substituted fluorene oligomers or polymers correspond to Formula V, 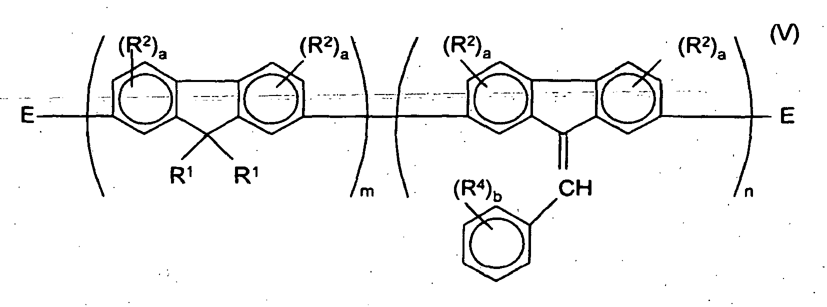 wherein:
E, R¹' R², a, m and n are as defined hereinbefore;
R⁴ is independently in each occurrence di(C₁₋₂₀ alkyl) amino, C₁₋₂₀ hydrocarbyloxy, tri(C₁₋₁₀ alkyl)siloxy or C₁₋₂₀ hydrocarbyl; and
b is independently in each occurrence a number of from 0 to 3, preferably no greater than 2, and most preferably no greater than 1.

R⁴ is preferably di (C₁₋₁₂ alkyl) amino, C₁₋₁₀ alkoxy, C₆₋₁₀ aryloxy or alkyl-substituted aryloxy, tri(C₁₋₄ alkyl)-siloxy, C₁₋₁₀ alkyl, or C₆₋₁₀ aryl or alkyl-substituted aryl. Even more preferably, R⁴ is di(C₁₋₆ alkyl)amino, C₁₋₁₀ alkoxy or C₁₋₁₀ alkyl. In another embodiment, preferably C₁₋₁₀ alkyl, C₆₋₁₀ aryl or C₇₋₁₀ alkyl aryl and more preferably C₁₋₁₀ alkyl. Preferably, b is 0 to 2, and most preferably b is 1.

In one preferred embodiment, the aryl moieties at the terminal 2- and 7- or 7'-position (terminal position) have acrylate and methacrylate ester reactive groups preferably corresponding to Formula VI. 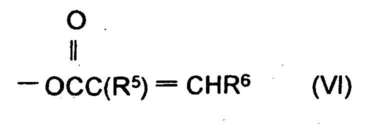 wherein:
R⁵ is hydrogen or C₁₋₄ alkyl, preferably hydrogen or methyl, and
R⁶ is hydrogen, C₁₋₂₀ hydrocarbyl or C₁₋₂₀ hydrocarbyloxy, preferably hydrogen or C₁₋₂₀ hydrocarbyl.

More preferably, R⁶ is hydrogen, C₁₋₁₀ alkyl or C₆₋₁₀ aryl or alkyl-substituted aryl. Even more preferably, R⁶ is hydrogen, C₁₋₄ alkyl or phenyl. Most preferably, R⁶ is hydrogen.

In another preferred embodiment, ethenyl, and aryl moieties at the terminal position correspond to Formula VII,

- C(R⁷) = C(R⁷)H (VII)

wherein:
R⁷ is independently in each occurrence hydrogen, C₁₋₂₀ hydrocarbyl or C₁₋₂₀ hydrocarbyloxy. Preferably, R⁷ is hydrogen, C₁₋₁₀ alkyl, C₆₋₁₀ aryl or alkyl-substituted aryl or C₁₋₂₆ alkoxy. More preferably, R⁷ is hydrogen, C₁₋₄ alkyl, phenyl or C₁₋₄ alkoxy.

In one embodiment, the E is a benzocyclobutene moiety which preferably corresponds to Formula VIII, 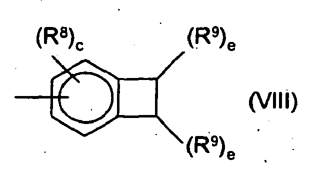 wherein:
R⁸ is C₁₋₂₀ alkyl, C₁₋₂₀ alkoxy, C₁₋₂₀ alkylthio, C₆₋₂₀ aryl, C₆₋₂₀ aryloxy, C₆₋₂₀ arylthio, C₇₋₂₀ aralkoxy, C₇₋₂₀ alkaryloxy, C₇₋₂₀ alkarylthio, C₇₋₂₀ aralkyl, C₇₋₂₀ aralkoxy, C₇₋₂₀ aralkylthio, cyano, carboxylate, C₁₋₂₀ hydrocarbylcarbonyloxy, C₁₋₂₀ hydrocarbylsulfinyl, C₁₋₂₀ hydrocarbylsulfonyl, amino or C₁₋₂₀ dialkylamino;
R⁹ is cyano, carboxylate, C₁₋₂₀ hydrocarbylcarbonyloxy, nitro, halo, C₁₋₂₀ hydrocarbylsulfonyl, C₁₋₂₀ hydrocarbylsulfinyl, C₁₋₂₀ alkyl, amido or C₁₋₂₀ hydrocarbyloxy' preferably C₁₋₂₀ hydrocarbyloxy or cyano;
c is 0 or an integer of 1 to 3, preferably from 0 to 1 and most preferably 0; and
e is 0 or 1.

As used herein 2,7-aryl-9-substituted fluorenes include compounds where either of m or n is 1 and the other is 0.

In one preferred embodiment, the invention comprises 2,7-aryl-9,9-dihydrocarbyl- or cyclohydrocarbdiylfluorenes and 9,9-dihydrocarbyl- or cyclohydrocarbdiylfluorene oligomers and polymers which correspond to Formula IX, 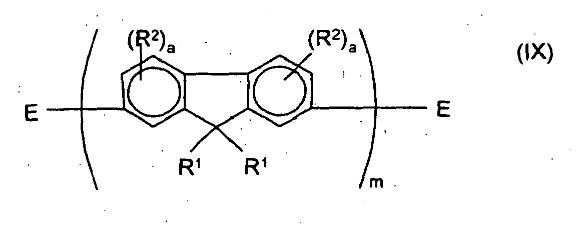 wherein E, R¹, R², a and m are as described hereinbefore.

In another embodiment, the invention comprises 2,7-aryl-9-hydrocarbylidenylfluorenes and 9-hydrocarbylidenylfluorene oligomers and polymers thereof which preferably correspond to Formula X, 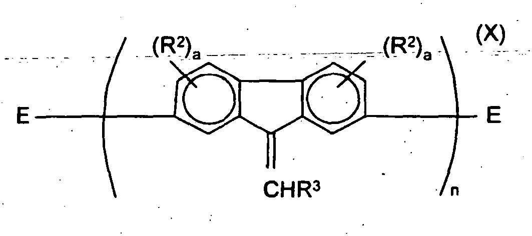 wherein E, R², R³, a and n are as described hereinbefore, and n is at least 1.

In one preferred embodiment, the 2,7-aryl-9-hydrocarbylidenylfluorenes and 9-hydrocarbylidenylfluorene oligomers and polymers are 2,7-aryl-9-benzylidenylfluorenes and 9-benzylidenylfluorene_oligomers and polymers which correspond to Formula XI, 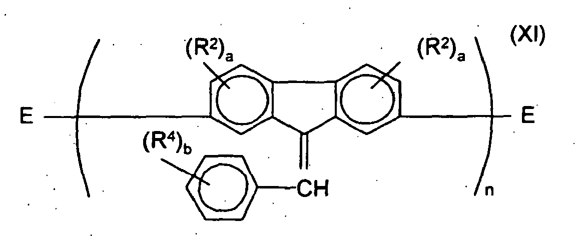 wherein E, R², R⁴, a, b and n are as described hereinbefore, and n is at least 1.

As used herein, 2,7-aryl-9-substituted fluorenes include compounds corresponding to Formulas IX, X or XI where m or n is 1. As used herein, 9-substituted fluorene oligomers and polymers correspond to Formulas IX, X or XI wherein m or n is greater than 1 and Formulas IV and V where both m and n are 1 or greater.

The fluorenes and fluorene oligomers or polymers of the invention demonstrate strong photoluminescence in dilute solutions or in the solid state. When such materials are exposed to a light of a wavelength of 300 to 700 nanometers, the materials emit light of wavelengths in the region of 400 to 800 nanometers. More preferably, such materials-absorb light of wavelengths of from 350 to 400 nanometers and emit light of wavelengths in the region of 400 to 650 nanometers. The fluorenes and fluorene oligomers or polymers of the invention are readily soluble in common organic solvents. They are processable into thin films or coatings by conventional techniques. Upon curing, such films demonstrate resistance to common organic solvents and high heat resistance. Generally, the fluorene oligomers and polymers are liquid crystalline in nature. The fluorenes and fluorene oligomers or polymers of the invention are capable of crosslinking to form solvent resistant, heat resistant films at 100°C or more, more preferably at 150°C or more. Preferably, such crosslinking occurs at 350°C or less, more preferably 300°C or less and most preferably 250°C or less. The fluorenes and fluorene oligomers or polymers of the invention are stable at 100°C or more and more preferably 150°C or more. Stable, as used herein, means that such monomers or oligomers do not undergo crosslinking or polymerization reactions at or below the stated temperatures.

The fluorene oligomers or polymers of this invention preferably have a weight average molecular weight of 1000 Daltons or greater, more preferably 5000 Daltons or greater, even more preferably 10,000 Daltons or greater, even more preferably 15,000 Daltons or greater and most preferably 20,000 Daltons or greater; preferably 1,000,000 Daltons or less, more preferably 500,000 Daltons or less and most preferably 100,000 Daltons or less. Molecular weights are determined according to gel permeation chromatography using polystyrene standards.

The 9 substituted fluorene oligomers or polymers demonstrate a polydispersity (Mw/Mn) of less than 5, more preferably 4 or less, even more preferably 3 or less, even more preferably 2.5 or less and most preferably 2.0 or less.

"Hydrocarbyl" as used herein shall mean any organic moiety containing only hydrogen and carbon unless specified otherwise, and may include aromatic, aliphatic, cycloaliphatic and moieties containing two or more of aliphatic, cycloaliphatic and aromatic moieties.

The 2,7-dihalo-9,9-dihydrocarbyl-fluorenes may be prepared by reacting a 2,7-dihalofluorene with at least 2 moles of hydrocarbyl halide in the presence of a phase transfer catalyst and an alkali metal hydroxide. One embodiment of this process is described in Equation 1: 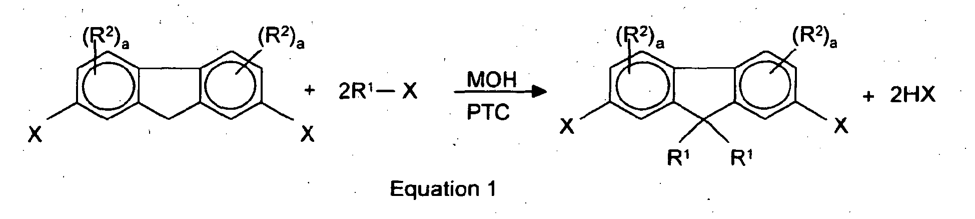 wherein R¹, R², X and a are as previously defined, and M is an alkali metal and PTC is phase transfer catalyst.

Preferred hydrocarbyl halides are C₃₋₁₂ alkyl halides and C₆₋₁₀ aryl or alkaryl halides. More preferable are the C₃₋₁₂ alkyl halides.

The hydrocarbyl halide is contacted with the 2,7-dihalofluorene in a mole ratio such that a high yield of 2,7-dihalo-9,9-dihydrocarbylfluorene is prepared. Preferably, the mole ratio of hydrocarbyl halide to 2,7-dihalofluorene is 2:1 or greater, more preferably 2.2:1 or greater and even more preferably 3:1 or greater. Preferably, the mole ratio of hydrocarbyl halide to 2,7-dihalofluorene is 6:1 or less, more preferably 5:1 or less and most preferably 4:1 or less.

The process is performed in the presence of an alkali metal hydroxide in a sufficient amount to facilitate the efficient reaction of the hydrocarbyl halide or hydrocarbyl dihalide with the 2,7-dihalofluorene. Preferably, 2 equivalents or greater of alkali metal hydroxide is used in relation to 2,7-dihalofluorene and, more preferably 3 equivalents or greater of alkali metal hydroxide. Preferably, 20 equivalents or less of alkali metal hydroxide per equivalent of 2,7-dihalofluorene are used, more preferably 8 equivalents or less and most preferably 4 equivalents or less. Preferred alkali metal hydroxides useful are sodium hydroxide and potassium hydroxide, with sodium hydroxide being most preferred.

This process is an interfacial process using phase transfer catalysts. Any phase transfer catalyst known to those skilled in the art may be used. A sufficient amount of such phase transfer catalyst to facilitate the reaction of the hydrocarbyl halide or hydrocarbyl dihalide with the 2,7-dihalofluorene in a reasonably efficient manner is used. Preferable phase transfer catalysts include quaternary ammonium salts, quaternary phosphonium salts, polyethylene glycols and crown ethers. More preferred phase transfer catalysts are the quaternary ammonium salts. Examples of preferred quaternary ammonium salts useful as phase transfer catalysts include benzyltrimethylammonium chloride, benzyltriethylammonium chloride and tetrabutylammonium bromide. The phase transfer catalysts preferably are used in an amount of 0.0001 mole or greater of catalyst per mole of 2,7-dihalofluorene, more preferably 0.001 mole or greater and even more preferably 0.01 mole or greater. Preferably, 0.2 mole or less of catalyst per mole of 2,7-dihalofluorene is used, more preferably 0.15 mole or less and even more preferably 0.02 mole or less.

The 2,7-dihalo-9,9-cyclohydrocarbdiylfluorene may be prepared by contacting 1 mole of 2,7-dihalofluorene with 1 mole of hydrocarbyl dihalide in the presence of a catalytic amount of a tetraalkylammonium hydroxide.

This process is described in Equation 2: 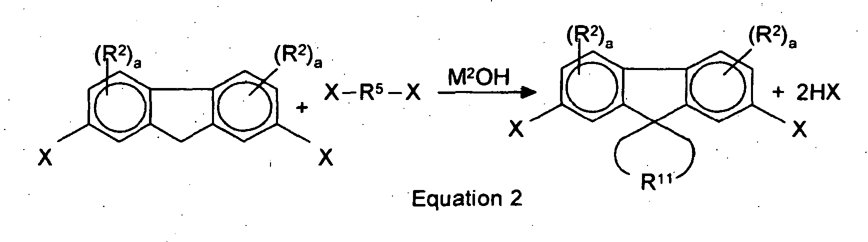 wherein:
R² X and a are as previously defined;
M² is a tetraalkylammonium moiety; and
R¹¹ is a C₅₋₂₀ straight- or branched-chain aliphatic divalent moiety or C₄₋₂₀ straight- or branched-chain aliphatic divalent moiety containing one or more heteroatoms selected from S, N and O.

Preferred hydrocarbyl dihalides are C₄₋₂₀ straight- or branched-chain aliphatic dihalides or C₃₋₂₀ straight- or branched-chain aliphatic dihalides containing one or more heteroatoms selected from S, N and O. More preferred hydrocarbyl dihalides are C₄₋₁₀ aliphatic dihalides or C₃₋₁₀ aliphatic dihalides containing one or more of S or O. Even more preferred hydrocarbyl dihalides are C₃₋₁₀ alkyl dihalides and C₃₋₁₀ alkyl ether dihalides (C₃₋₁₀ alkyl dihalides containing an oxygen). R¹¹ is more preferably a C₄₋₁₀ aliphatic divalent moiety or a C₃₋₁₀ aliphatic divalent moiety containing one or more of S or O. R¹¹ is even more preferably a divalent C₄₋₁₀ alkyl and C₃₋₁₀ alkyl ether (C₃₋₁₀ alkdiyl containing an oxygen). Preferably, hydrocarbyl halides are used in this process.

The 2,7-dihalo-9-hydrocarbylidene fluorenes of the invention may be prepared by the reaction of a 2,7-dihalo-fluorine with a hydrocarbylaldehyde or a substituted hydrocarbylaldehyde in the presence of base as a catalyst. Preferably, the aldehyde corresponds to the formula 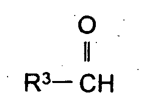 wherein R³ is as defined previously. In a more preferred embodiment, the hydrocarbyl moiety is a phenyl, a substituted phenyl, a C₃₋₁₀ aliphatic moiety or a C₅₋₁₀ cycloaliphatic moiety and the aldehyde is benzaldehyde, substituted benzaldehyde, C₃₋₁₀ aliphatic aldehyde or C₅₋₁₀ cycloaliphatic aldehyde. This reaction is illustrated by Equation 3: 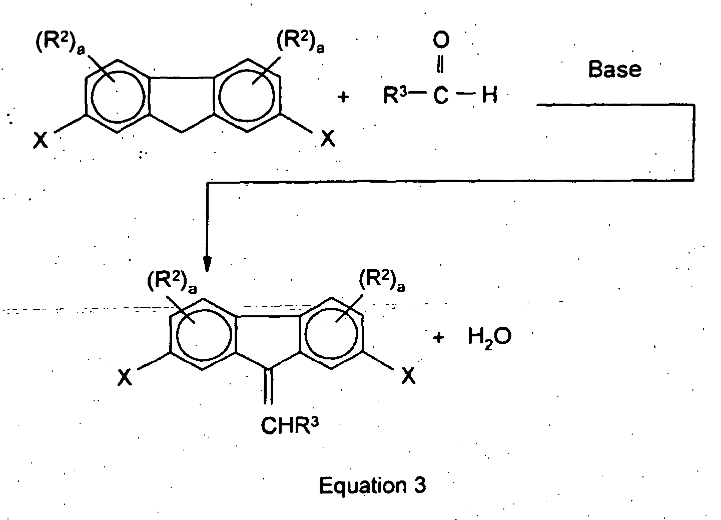 wherein X, R², R³ and a are as previously defined.

The 2,7-dihalofluorene is reacted with a sufficient amount of hydrocarbylaldehyde to prepare the hydrocarbylidene-substituted 2,7-dihalofluorenes in high yield. Preferably, the mole ratio of hydrocarbylaldehyde to 2,7-dihalofluorene is 1.0 or greater, more preferably 1.5 or greater and even more preferably 2 or greater. Preferably, the mole ratio of hydrocarbylaldehyde to 2,7-dihalofluorene is 6 or less, more preferably 3 or less and most preferably 2 or less.

The 2,7-dihalo-9,9-bis-C₄₋₁₆ hydrocarbyl carbonyloxy-substituted 2,7-dihalofluorenes may be prepared by base catalyzed addition of 2,7-dihalofluorene to alkyl acrylates and alkyl methacrylates as described in US-A-3,641,115.

The 2,7-dihalo C₆₋₁₆ aryl (trialkylsiloxy)-substituted fluorenes may be prepared by the following process. 2,7-dihalofluorenone is reacted with phenol in a mixture of methanesulfonic acid and 3-mercaptopropionic acid to provide 2,7-dihalo-9,9-bis-(4-hydroxyphenyl)fluorene which is then treated with a trialkylsilyl chloride in the presence of a base to yield the 2,7-dihalo-9,9-bis(4-trialkylsiloxyphenyl) fluorene. 2,7-Dihalofluorenone can be prepared by the oxidation of 2,7-dihalofluorene with oxygen in the presence of a base, such as potassium t-butoxide, in t-butyl alcohol. The conditions for this process are disclosed in Yang, "Novel Carbon Catalysts: Oxidation in Basis Solution, "J. Organic Chemistry, Vol. 58, p. 3754 (1958). Alternatively, 2,7-dihalofluorene can be oxidized to 2,7-dihalofluorenone by contacting it with chromium oxide (CrO₃ in acetic acid according to the process disclosed in Hodgkinson et al., J. Chem. Soc., Vol. 43, pp. 163-172 (1983). The 2,7-dihalofluorenone is contacted with 3 to 10 equivalents of phenol in the presence of from 30 to 100 percent by weight of methanesulfonic acid and from 2 to 10 percent by weight of mercaptopropionic acid. The reaction is preferably performed at a temperature of from 20°C to 50°C. The 4-hydroxyphenyldihalofluorene is recovered by conventional techniques before reaction with the trialkylsilyl chloride.

The 2,7-dihalo-9,9-bis(4-hydroxyphenyl) fluorene is contacted with from 2.2 to 3.0 equivalents of trialkylsilyl chloride in the presence of from 3.0 to 6.0 equivalents of base. The reaction is preferably performed at a temperature of from 20°C to 40°C. The reaction is performed in a solvent of dimethylformamide and dimethylacetamide. Imidazole is the preferred base. The 2,7-dihalo-9,9-bis(4-trialkylsiloxyphenyl)fluorene can be recovered by conventional techniques.

The 2,7-dihalo-9-substituted fluorenes may be further substituted on the 3-, 4-, 5- and/or 6-position by any suitable synthesis technique. Preferably, the 3-, 4-, 5- and/or 6-positions are substituted prior to substitution at the 9-position. In many instances, the reaction sequence to place substituents at the 3-, 4-, 5- and/or 6-position may result in unwanted substitution on the substituents at the 9-position if the substitution is performed after the 9-position substitution.

The 2,7-aryl-9-substituted fluorene oligomers and polymers of the invention are prepared by contacting one or more 2,7-dihalo-9-substituted fluorenes with a haloaromatic compound in the presence of a nickel (zero valent) catalyst or other catalysts suitable for use in processes for the de-halogen-coupiing of aryl halides. The 9-substituted fluorene oligomers and polymers terminated at the terminal 2- and 7'-positions with hydrogen or a halogen may be prepared by coupling the 2,7,-dihalo-9-subsituted fluorenes to each other in a suitable process for the coupling of aryl halides. The nickel (zero valent) catalyst may be prepared *in situ* by contacting a divalent nickel salt with a reducing agent in the presence of a material capable of acting as a ligand and optionally a material capable of accelerating the reactions. Preferably, the 2,7-dihalo-9-substituted fluorene corresponds to Formulas XII or XIII (same as Formulas I and II respectively), 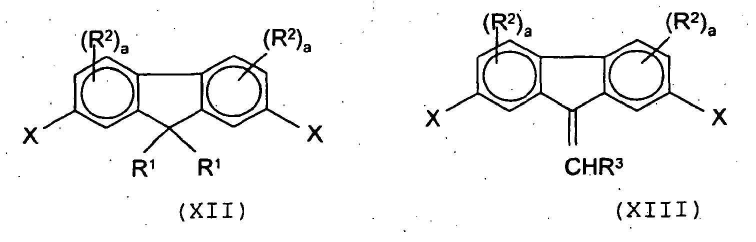 wherein R¹, R², R³ and a are as previously defined and X is a halogen moiety. In Formulas XII and XIII, X is preferably bromine or chlorine.

The 2,7-dihalo-9-substituted fluorenes and 2,7-dihalo-9-hydrocarbylidenylfluorenes are prepared from 2,7-dihalofluorene, which is commercially available, by the processes described herein.

The haloaromatic compound used to prepare the 2,7-aryl-9-substituted fluorenes, and 9-substituted fluorene oligomers and polymers comprises an aromatic compound substituted on the ring with a halogen and may further be substituted with a moiety capable of crosslinking or chain extension. Preferably, such compound corresponds to the formula X-Ar, wherein X is as previously defined and Ar is an aryl moiety or an aryl moiety substituted with a moiety capable of crosslinking or chain extension or a trialkylsiloxy moiety.

Preferably, the haloaromatic compound corresponds to Formulas XIV or XV, 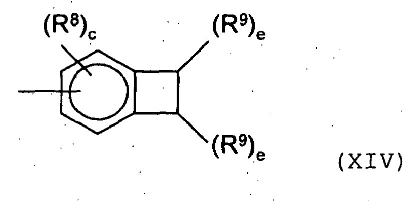 or 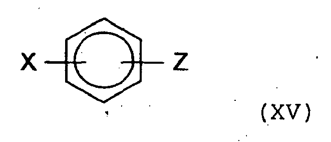 wherein:
R⁸, R⁹, c and e are as defined above and
Z is a trialkylsiloxy, glycidyl ether, acrylate ester, methacrylate ester, ethenyl, ethynyl, maleimide or a trifluorovinyl ether moiety, nadimide, or hydroxy. Preferably, Z is a trialkylsiloxy moiety, ethenyl, ethynyl, maleimide or trifluorovinyl ether moiety. More preferably, Z is a trialkylsiloxy moiety.

In one preferred embodiment, the haloaromatic compound is a halogen-substituted benzocyclobutene moiety according to Formula XIV.

The 2,7-dihalo-9-substituted fluorenes of the invention are useful in preparing oligomers which can be used to form films. Such films can be used in polymeric light-emitting diodes. Preferably, such films are used as emitting layers. The oligomers of 2,7-dihalo-9-substituted fluorenes are prepared by reacting 2,7-dihalo-9-substituted fluorenes with a haloaromatic, such as a halobenzene, further substituted with a reactive end-group such as ethenyl, ethynyl, maleimide, nadimide, trifluorovinyl ether or cyclobutene ring. Such reaction is performed using a nickel-zinc coupling reaction wherein the degree of oligomerization can be controlled by the ratio of reactants.

The preparation of the 2,7-diaryl-9-substituted fluorenes and the 9-substituted fluorene oligomers or polymers may be illustrated by Equations 4 and 5.

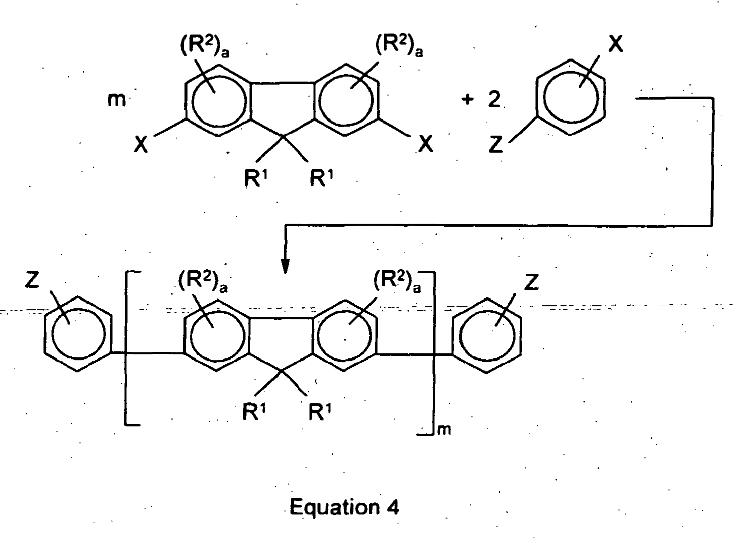

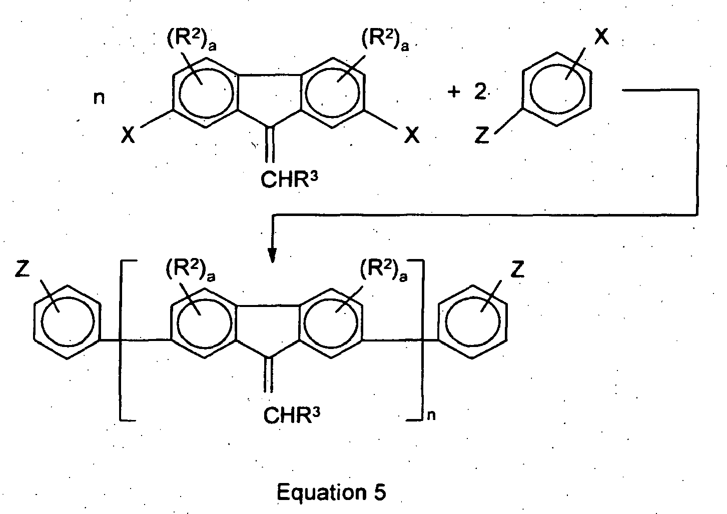

The 2,7-dihalo-9-substituted fluorene and haloaromatic compound may be contacted in a wide range of ratios, depending upon the desired degree of oligomerization or polymerization. Preferably, the mole ratio of 2,7-dihalo-9-substituted fluorene to haloaromatic compound is 1:2 (i.e. 0.5:1) or greater, preferably 1:1 or greater and more preferably 2:1 or greater. Preferably, the ratio is 50:1 or less, and more preferably 25:1 or less.

In the embodiment wherein it is desired to prepare a 2,7-diaryl-9-substituted fluorene (where n is 1), the mole ratio of 2,7-dihalo-9-substituted fluorene to haloaromatic compound is 1:2. In the embodiment where oligomers or polymers are desired (where m+n is greater than 1), a greater ratio of 2,7-dihalo-9-substituted fluorene is used relative to the haloaromatic compound. Higher ratios facilitate the preparation of higher molecular weight oligomers and polymers.

In a preferred embodiment, the reaction of the 2,7-dihalo-9-substituted fluorene with haloaromatic compound takes place according to the procedures of Colon et al., described in Journal of Polymer Science, Part A, Polymer Chemistry Edition, Vol. 28, p. 367 (1990), incorporated herein by reference, and Colon et al., Journal of Organic Chemistry, Vol. 51, p. 2627 (1986).

The resulting 2,7-diaryl-9-substituted fluorenes and 9-substituted fluorene oligomers and polymers are recovered according to conventional techniques; preferred techniques include filtration and precipitation using a nonsolvent. Alternatively, in another embodiment, the 2,7-diaryl-9-substituted fluorenes and 9-substituted fluorene oligomers and polymers may be prepared by a process disclosed in Ioyda et al., Bulletin of the Chemical Society of Japan, Vol. 63, p. 80 (1990). Such method is similar to the method described hereinbefore. In particular, the catalyst is a divalent nickel salt introduced as a nickel halide bis-triphenylphosphine complex. The reaction may be performed in a variety of solvents including acetone, dimethylformamide, tetrahydrofuran and acetonitrile. The reaction is accelerated by the addition of 10 mole percent of an organo-soluble iodide such as tetraethylammonium iodide. Such a reaction is performed at a temperature of from 20°C to 100°C for 1 to 24 hours.

In another embodiment, the oligomers and polymers may be prepared via the processes disclosed by Yamamotto, Progress in Polymer Science, Vol. 17, p. 1153 (1992). In such process, 2,7-dihalo-9-substituted fluorene monomers are contacted with at least a stoichiometric amount of nickel catalyst in the form of nickel (1,5-cyclooctadiene) complex and at least a stoichiometric amount of 1,5-cyclooctadiene as a ligand in an inert solvent, such as tetrahydrofuran. The reaction is preferably conducted at 70°C or higher for 2 or more days. In another embodiment, the subject compounds of the invention may be prepared by the process disclosed in Miyaura et al., Synthetic Communication, Vol. 11, p. 513 (1981) and Wallow et al., American Chemical Society Polymer Preprint Vol. 34, (1), p. 1009 (1993). In such process, 2,7-dihalo-9-substituted fluorenes are converted to the corresponding diboronic acid by reacting the 2,7-dilithio- or 2,7-diGrignard-9-substituted fluorenes with trialkyl borates. M. Rehalin et al., as disclosed in Makromoleculaire Chemie, Vol. 191, pp. 1991-2003 (1990). The diboronic acid is then reacted with a 2,7-dihaloflourene in the presence of a catalytic amount of tetrakistriphenylphosphine palladium and an aqueous base at 70°C or higher for 10 to 100 hours in an inert solvent, for instance toluene and ethanol.

In the embodiment wherein the reactive moiety on the aryl moieties of the 2,7-aryl-9-substituted fluorene or 2,7'-aryl-9-substituted fluorene oligomers and polymers are trialkylsiloxy moieties, the trialkylsiloxy moieties may be converted to hydroxy moieties by contact with concentrated acid, such as hydrochloric acid, in an organic solvent.

A halophenyl trialkylsiloxy ether can be reacted with the 2,7-dihalo-9-substituted fluorene to prepare an oligomer having end-groups comprising phenyl trialkylsiloxy moieties. This reaction sequence is illustrated by Equation 6 wherein R¹⁰ is a C₁₋₂₀ alkyl moiety, preferably a C₁₋₄ alkyl moiety. The trialkylsiloxy moieties can be converted to hydroxy moieties by refluxing the resulting product in tetrahydrofuran and concentrated hydrochloric acid. This reaction sequence is illustrated by Equation 7.

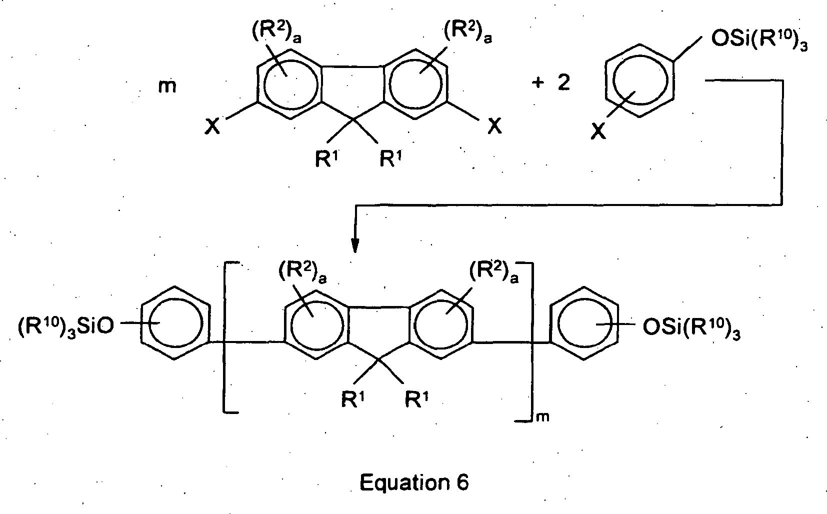

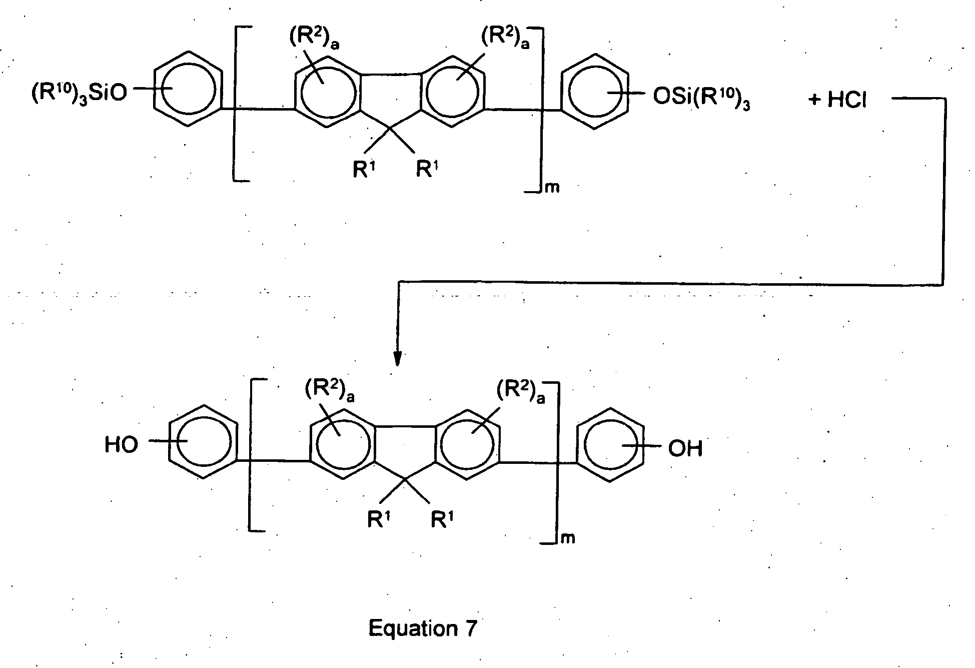

The hydroxy moieties of the 2,7'-aryl substituents may be converted to cyanate moieties by well-known cyanation reactions. See, for example, US-A-4,478,270; Martin, Organic Synthesis, Vol. 61, p. 35; and Handbook of Preparative Inorganic Chemistry, p. 1,662 (1963), Academic Press, New York. This reaction sequence is illustrated by Equation 8.

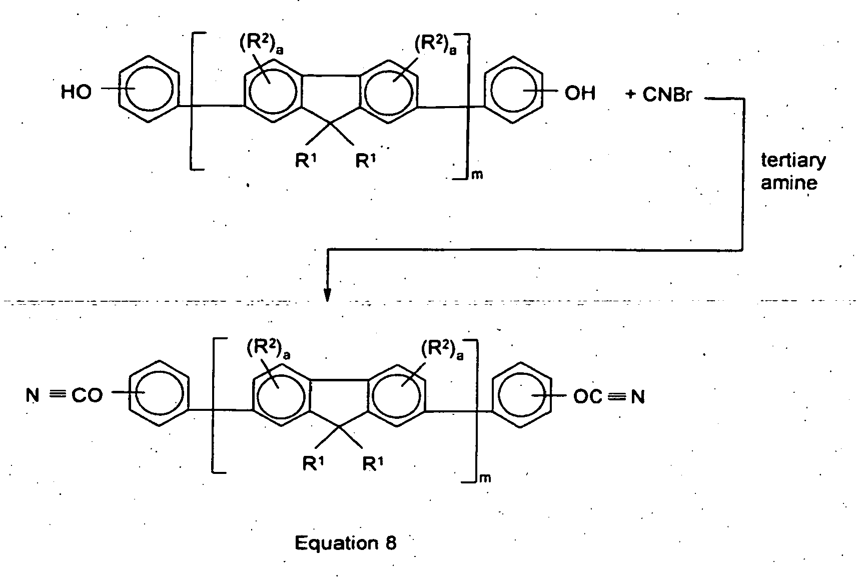

In one preferred embodiment, the 2,7-hydroxyaryl-9-substituted fluorene or 2,7'-hydroxyaryl-9-substituted fluorene oligomer or polymer is contacted with cyanogen halide dissolved in a chlorinated hydrocarbon or a secondary or tertiary alcohol, in the presence of a tertiary amine at a temperature of 0°C or less under conditions such that the hydroxy moieties are replaced with cyanate moieties. Preferably, the contacting occurs in the presence of a dilute base such as alkali or alkaline metal hydroxides, alkali or alkaline metal carbonates, alkali or alkaline metal bicarbonates or tertiary amines. Preferred bases are the tertiary amines with the aliphatic tertiary amines being most preferred. This process is preferably run at a temperature of 0°C or lower with temperatures of -10°C or lower being most preferred. It is preferable to perform such process under an inert gas atmosphere. The cyanated 2,7-aryl-9-substituted fluorenes or 2,7'-aryl-9-substituted fluorene oligomers or polymers may be recovered by washing the reaction solution with a dilute base to remove excess cyanogen chloride. The reaction solution is thereafter washed with water so as to remove any salt prepared from the hydrochloride by-product and base. The reaction solution is then contacted with the dilute acid to neutralize any base which may be present. Thereafter, the reaction solution is contacted with water again so as to remove any other impurities and the cyanated 2,7-aryl-substituted-9-substituted fluorenes and 2,7'-aryl-9-substituted fluorene oligomers or polymers are recovered by drying the solution with the use of a dessicant.

The reactions illustrated by Equations 6, 7 and 8 can also be performed starting with 9-hydrocarbylidenyl-2,7-dihalofluorene.

In another embodiment, the hydroxy moieties of the 2,7-hydroxyaryl-9-substituted fluorene or 2,7'-hydroxy-aryl-9-substituted fluorene oligomer or polymer may be converted to glycidyl ether moieties by processes well known in the art. Such glycidyl ethers are preferably prepared by contacting the 2,7-hydroxyaryl-9-substituted fluorene or 2,7'-hydroxyaryl-9-substituted fluorene oligomer or polymer with epihalohydrin under conditions to form aryl moieties with chlorohydrin groups at their termini. The chlorohydrin groups are dehydrohalogenated to form an epoxy or glycidyl ring by contacting them with sodium hydroxide. Such process is described in Handbook of Epoxy Resins, Lee and Neville (1967). This process is illustrated by Equation 9.

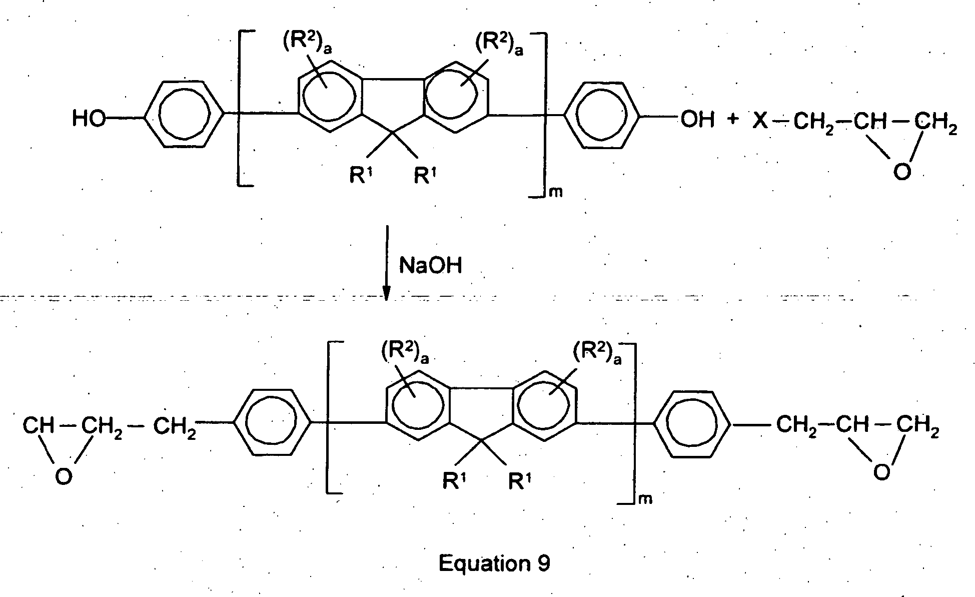

The 2,7-diaryl-9-substituted fluorenes and 9-substituted fluorene oligomers and polymers are useful in preparing coatings and films. Such coatings and films can be useful as emitting layers in polymeric light-emitting diodes, in protective coatings for electronic devices and as fluorescent coatings. The thickness of the coating or film is dependent upon the ultimate use. Generally, such thickness can be from .01 to 200 micrometers. In that embodiment wherein the coating is used as a fluorescent coating, the coating or film thickness is from 50 to 200 micrometers. In that embodiment where the coatings are used as electronic protective layers, the thickness of the coating can be from 5 to 20 micrometers. In that embodiment where the coatings are used in a polymeric light-emitting diode, the thickness of the layer formed is 0.05 to 2 micrometers. The compounds of the invention and their oligomers or polymers form good pinhole- and defect-free films. Such films can be prepared by means well known in the art including spin-coating, spray-coating, dip-coating and roller-coating. Such coatings are prepared by a process comprising applying a composition to a substrate and exposing the applied composition to conditions such that a film is formed. The conditions which form a film depend upon the application technique and the reactive end groups of the aryl moiety. In a preferred embodiment, the composition applied to the substrate comprises the 2,7-diaryl-9-substituted fluorene or 9-substituted fluorene oligomers or polymers dissolved in a common organic solvent. Preferred solvents are aliphatic hydrocarbons, chlorinated hydrocarbons, aromatic hydrocarbons, ketones and ethers. It is preferable that such solvents have relatively low polarity. Preferably, the solution contains from 0.1 to 10 weight percent of the 2,7-diaryl-9-substituted fluorene or 9-substituted fluorene oligomers or polymers. For thin coatings, it is preferred that the composition contains from 0.5 to 5.0 percent by weight of the 2,7-diaryl-9-substituted fluorene or 9-substituted fluorene oligomers or polymers. This composition is then applied to the appropriate substrate by the desired method and the solvent is allowed to evaporate. Residual solvent may be removed by vacuum and/or by heat. If the solvent is low boiling, then low solution concentrations, for example, 0.1 to 2 percent, are desired. If the solvent is high boiling, then high concentrations, for example, 3 to 10 percent, are desired. After removal of the solvent, the coating is then exposed to the necessary conditions to cure the film, if needed, to prepare a film having high solvent and heat resistance. The films are preferably substantially uniform in thickness and substantially free of pinholes. Preferably, the films are cured when exposed to temperatures of 100°C or greater, more preferably 150°C or greater and most preferably 200°C or greater. Preferably, the films cure at a temperature of 300°C or less.

In the preparation of the films, the composition may further comprise a catalyst suitable to facilitate or initiate the curing of the films. Such catalysts are well known in the art, for instance, for materials having ethylenic unsaturation, a free radical catalyst may be used. For aryl moieties with glycidyl ethers as end-groups, ureas or imidazoles may be used. In the preparation of films from fluorenes with glycidyl ether aryl-terminal moieties, such materials may be reacted with commonly known curing agents which facilitate cross-linking. Among preferred curing agents are tetrahydrophthalic anhydride, nadic anhydride and maleic anhydride.

In another embodiment the 2,7-diaryl-9-substituted fluorenes and 9-substituted fluorene oligomers or polymers may be partially cured. This is known as B-staging. In such embodiment, the fluorenes and their oligomers or polymers thereof are exposed to conditions such that a portion of the reactive materials cure and a portion of the reactive materials do not cure. This is commonly used to improve the handleability of such a resin and can facilitate the preparation of the films. Such B-staged material can thereafter be used to prepare coatings by the means disclosed hereinbefore. Preferably, 10 mole percent or greater of the reactive moieties are reacted. Preferably, 50 mole percent or less of the reactive moieties are reacted.

The following examples are included for illustrative purposes only and do not limit the scope of the claims. Unless otherwise stated, all parts and percentages are by weight.

### Example 1 - 2,7'-Dichloro-9,9-di(2ethylhexyl)fluorene

To a stirred mixture of 2,7-dichlorofluorene (43 g, 0.183 mole) and 120 mL of dimethylsulfoxide (DMSO) under nitrogen was added benzyltriethylammonium chloride (2.3 g, 0.01 mole) and 60 mL of a 50 weight percent aqueous solution of sodium hydroxide. 2-Ethylhexyl bromide (85 g, 0.44 mole) was added and the mixture was agitated well for 2 hours. The reaction was exothermic, the temperature reaching 80°C within 5 minutes after the addition of 2-ethylhexyl bromide and then falling off to 30°C over the 2-hour reaction time. Analysis of an aliquot by high pressure liquid chromatography (HPLC) showed the complete disappearance of 2,7-dichlorofluorene and the formation of new product. Water (200 mL), and diethyl ether (250 mL) were added to the reaction mixture, stirred for 15 minutes and the layers were separated. The organic layer was washed with a saturated aqueous NaCl solution, water, dried (MgSO₄) and evaporated to remove ether. Fractional vacuum distillation of the residue provided 2,7-dichloro-9,9-di(2-ethylhexyl)fluorene as clear liquid, boiling point 200°C/l mm Hg (133 Pa), (79 g, 94 percent yield); HPLC analysis showed that the product was 99 percent pure; Proton Magnetic Spectrum (PMR) analysis was consistent with the title structure.

### Example 2 - 9,9-Di-n-butyl-2,7-dibromofluorene

2,7-Dibromofluorene (32.4 g, 0.1 mole), n-butyl bromide (75 g, 0.55 mole), tetra-n-butylammonium chloride (1.5 g) and 50 percent aqueous NaOH solution were stirred vigorously at 80°C for 1 hour. The reaction mixture was allowed to cool to room temperature and extracted with ether. The ether extracts were washed with water and dried over anhydrous magnesium sulfate. Removal of solvent gave a yellow solid which was recrystallized from 400 mL of ethanol to provide 9,9-di-n-butyl-2,7-dibromofluorene as colorless crystals (42 g, 96 percent yield), melting point 120.5°C to 121.5°C. HPLC analysis showed that the product had a purity of 99.5 percent and the proton and carbon-13 NMR were consistent with the title structure.

### Example 3 - 2,7-Dibromo-9,9-((2-methoxycarbonyl)-ethyl)fluorene

To a 500 mL, three-necked, round-bottomed flask equipped with a condenser, magnetic stirring bar, stopper and a rubber septum was added 2,7-dibromofluorene (70.0 g, 0.216 mole) and methyl acrylate (166.0 g, 1.9 mole). To that stirring mixture was added dropwise (via syringe) benzyltrimethylammonium methoxide (3.3 mL, 40 weight percent solution). An exotherm was noted after addition of a few drops; temperature rose to 60°C. After the addition was completed, the reaction was stirred for an additional 15 minutes.

Excess methyl acrylate was distilled off under reduced pressure. The crude product solidified after cooling to room temperature and was washed with hexane, then filtered. The crude solid was recrystallized from methanol to afford white crystals (79.0 g, 90 percent yield).

### Example 4 - 2,7-Dibromo-9,9-di(2-ethylhexyl)fluorene

2,7-Dibromo-9,9-di(2-ethylhexyl)fluorene, prepared from 2,7-dibromofluorene and 2-ethylhexylbromide by the procedure of Example 1, was obtained as a pale-yellow syrup in 93 percent yield after passing the crude product through a column of silica gel and eluting the product with hexane. The carbon and proton magnetic spectra were found to be consistent with the title structure.

### Example 5 - 2,7-Dichloro-9,9-di(3-methyl-1-butyl)-fluorene

Following the procedure of Example 1, 2,7-dichloro-9,9-di(3-methyl-1-butyl)fluorene was prepared from 2,7-dichlorofluorene and 1-bromo-3-methylbutane as colorless crystals (recrystallized from pentane) in 90 percent yield, melting point 116°C to 117.5°C. The spectral data were consistent with the title structure and HPLC analysis showed the purity as 99 percent and greater.

### Example 6 - 2,7-Dichloro-9,9-di(1-hexyl)fluorene

2,7-Dichloro-9, 9-di(1-hexyl)fluorene was prepared from 2,7-dichlorofluorene and 1-bromohexane following the procedure of Example 1 as colorless crystals (recrystallized from hexane) in 91 percent yield; melting point 66°C to 67°C. HPLC analysis showed that the sample was 99 percent or greater pure and the spectral data confirmed the title structure.

### Example 7 - 2,7-Dichloro-9-benzylidenylfluorene

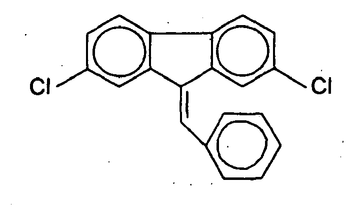

To a stirred suspension of 2,7-dichlorofluorene (6.4 g, 27 mmole) in 30 mL of pyrrolidine or pyridine at 0°C under nitrogen was added 6 mL of a 1M solution of tetrabutylammonium hydroxide in methanol. A solution of benzaldehyde (3.4 g, 32 mmole) in 25 mL of pyridine was then added over 10 minutes and the orange-colored mixture was allowed to stir at ambient temperature for 2 hours. The mixture was poured into 300 mL of ice-water, stirred for 3 hours, the yellow solid was filtered and recrystallized from n-heptane to provide 2,7-dichloro-9-benzylidenylfluorene as yellow needles, melting point 87°C to 88°C (7.8 g, 89.6 percent). PMR analysis was consistent with the title structure.

### Example 8 - 2,7-Dibromo-9-(4-dimethylamino-benzylidenyl)fluorene

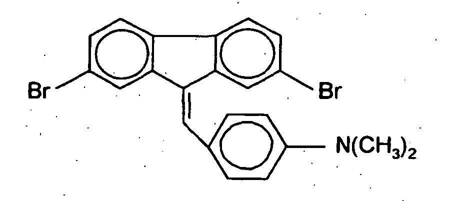

2,7-Dibromo-9-(4-dimethylaminobenzylidenyl)fluorene was prepared using the procedure of Example 1 in 87.7 percent yield as light-orange powder, melting point 214°C to 216°C (recrystallized from toluene). PMR analysis is consistent with the title structure.

### Example 9 - 2,7-Dibromo-9-(4-(2-ethylhexyloxy)-benzylidenyl)fluorene

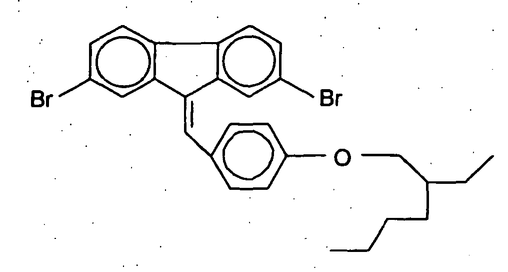

2,7-Dibromo-9-(4-(2-ethyl-hexyloxy) benzylidenyl)-fluorene was prepared from 2,7-dibromo-9-fluorene and 4-(2-ethylhexyloxyl)benzaldehyde using the procedure of Example 1 to give a yellow solid in 77 percent yield, melting point 68°C to 70°C. PMR analysis was consistent with the title structure.

### Example 10 - 2,7-Dibromo-9-(3,5,5-trimethyl-hexylidenyl)fluorene

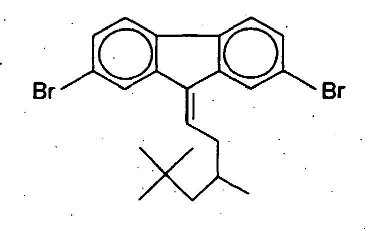

To a stirred mixture of 2,7-dibromofluorene (14 g, 40 mmole) and pyridine (40 mL) under nitrogen at -15°C was added 8 mL of a 1M solution of tetrabutylammonium hydroxide in methanol. A solution of 3,5,5-trimethyl-hexanal (7.6 g, 53 mmole) in pyridine was added dropwise and the reaction mixture was stirred at ambient temperature overnight. The mixture was poured into 600 mL of ice-water, stirred for 1 hour, the pale yellow solid was isolated, and recrystallized from ethanol to provide the title compound as pale yellow powder, melting point 108°C to 11.0°C (11.6 g, 64.7 percent). PMR analysis was consistent with the assigned structure.

### Example 11 - 2,7-Dibromo-9-(5-norborn-2-enylidenyl)-fluorene

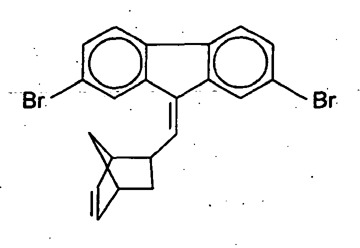

The title compound was prepared from 2,7-dibromofluorene and 5-norbornene-2-carboxaldehyde by following the procedure of Example 10. The product was extracted with ether and removal of ether gave a tan solid which was recrystallized from n-hexane to provide the title compound as tan crystals, melting point 118°C to 120°C (62 percent yield). PMR analysis and HPLC showed the title compound was obtained as a 1:1 mixture of exo and endo isomers.

### Example 12 - Poly(9,9-di-n-hexylfluorenyl-2,7'-diyl)

A dried polymerization reactor was charged with 2,7-dichloro-9,9-di-n-hexylfluorene (4.03 g, 10.0 mmoles), nickel chloride-2,2'-bipyridine complex (Ni complex) (43 mg, 0.15 mmole), zinc dust (1.96 g, 30 mmoles) and triphenylphosphine (TPP) (1.31 g, 5 mmoles). The reactor was evacuated and filled with nitrogen several times, and finally filled with argon. Dry dimethylacetamide (DMAc) (10 mL) was added and the contents of the reactor were stirred and heated in an 80°C oil bath. After 4 hours, a solid polymer cake was formed and the oil bath temperature was raised to 90°C. After 5.75 hours, 10 mL of dry toluene was added and stirring and heating were continued. Two more 10 mL portions of toluene were added at 6.5 hours and 7.3 hours. Heating and stirring were continued for 3.5 hours after the addition of the last portion of toluene. The mixture was poured into 150 mL of chloroform and filtered. The chloroform in the filtrate was removed on a rotary evaporator and the residue stirred with acetone. The large, bright-yellow granules obtained were dried for 18 hours in a vacuum oven at 70°C. The yield was 3.10 g, 93.9 percent. The polymer had the following chain structure:

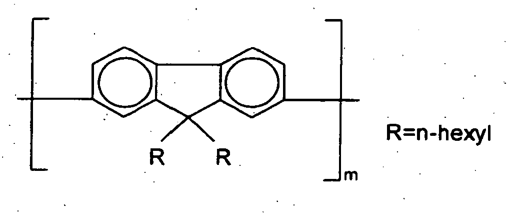

The inherent viscosity was 0.57 dL/g. Gel permeation chromatography showed Mw 39,630 Daltons, Mn 16,020 Daltons and polydispersity of 2.47. The degree of polymerization, m, was 48. Differential scanning calorimetry (DSC) analysis at 5°C/minute showed two endothermic transitions centered at 193°C and 249°C, indicative of liquid crystallinity. There was no indication of glass transition.

For comparison, Fukuda's polymer of formally the same chemical composition had a polydispersity of 6.8, degree of polymerization relative to polystyrene of 14, glass transition temperature of 55°C and no crystallinity.

### Example 13 - Poly(9,9-di-n-hexylfluorene-2,7'-diyl)

The experiment of Example 12 was repeated except the solvent was N-cyclohexylpyrrolidinone (20 mL). The polymerization was allowed to proceed for 18 hours and the product was purified and isolated as described in Example 12. The yield was 3.30 g, 100 percent. Inherent viscosity was 0.54 dL/g.

### Example 14 - Poly(9,9-di-n-hexylfluorene-2,7'-diyl)

A dried polymerization reactor was charged with nickel chloride (52 mg, 0.4 mmole), 2,2'-bipyridine (62.4 mg, 0.4 mole), zinc dust (5.76 g, 88 mmoles), and TPP (3.76 g, 14.0 mmoles). The reactor was evacuated and filled with nitrogen several times, and finally filled with argon. Dry DMAc (5 mL) was added and the content of the reactor stirred and heated in a 90°C oil bath for 15 minutes to generate the active Ni (0) catalyst. A degassed solution of 2,7-dichloro-9,9-di-n-hexylfluorene (8.06 g, 20.0 mmoles) in 17 mL of dry DMAc was then added in two portions via a syringe and the polymerization was allowed to proceed for 4.5 hours. The polymer isolated had inherent viscosity of 0.35 dL/g.

A thin film of the polymer was melted on a glass slide and examined on a hot-stage microscope under cross-polarized light. Intense birefringence was observed at above 200°C, indicating that the polymer undergoes a crystalline to liquid crystalline transition as suggested by DSC analysis.

### Example 15 - Photoluminescence of Poly(9,9-di-n-hexylfluorene-2,7'-diyl)

A dilute chloroform solution of the polymer prepared in Example 14 was spin-coated onto a quartz plate to give a dense, uniform film. Photoluminescence spectrum was obtained by excitation at 381 nm. The light emitted was intensely blue: major maximum at 424 nm with minor maxima at 445 and 475 nm. The emission spectrum reported by Fukuda consisted of a broad peak centered at 495 nm and a very minor peak at 425 nm. The differences in the emission spectra were consistent with the fact that Fukuda's polymer was substantially different from the polymer of this invention.

### Example 16 - Poly(9,9-di-(4-t-butyldimethylsilyloxyphenyl)fluorene-2,7'-diyl)

To a reactor was added nickel chloride (27 mg, 0.2 mmole), 2,2'-bipyridine (34 mg, 0.22 mmole), zinc dust (1.5 g, 23 mmoles) and TPP (2.62 g, 10 mmoles). The reactor was evacuated and filled with argon several times. Dry DMAc (30 mL) was added and the contents stirred and heated in a 70°C oil bath for 1 hour to generate the active catalyst. 2,7-Dibromo-9,9-di-(4-t-butyldimethylsilyloxyphenyl)fluorene (7.36 g, 10.0 mmoles) was added and the polymerization allowed to proceed for 23 hours. The solid mass was slurried with tetrahydrofuran (THF) and the slurry filtered to remove inorganic substances. The THF solution was stripped on a rotary evaporator and the residue extracted with ether. The ether-insoluble polymer was obtained as a yellow, granular solid and had inherent viscosity of 0.28 dL/g. Gel permeation chromatography analysis showed Mw 31,120 Daltons and Mn 20,970 Daltons and polydispersity of 1.48. The polymer had the following chain structure:

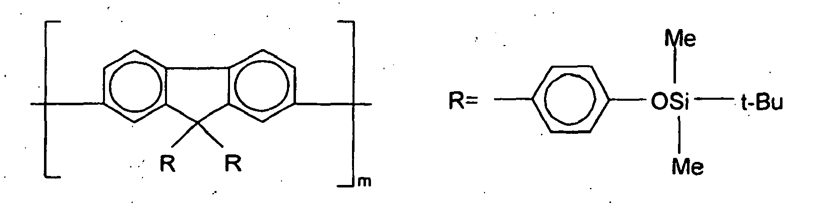

### Example 17 - Poly(9,9-di-(4-(2-ethylhexanoyloxy)-phenyl)fluorene-2,7'-diyl)

A reactor was charged with TPP (2.62 g, 10-mmoles), 2,7-dibromo-9,9-di-(4-(2-ethylhexanoyloxy)phenyl)fluorene (8.9 g, 11.7 mmoles), potassium iodide (0.22 g, 1.3 mmoles), DMAc (29 mL) and toluene (10 mL). Traces of moisture were removed as an azeotrope with toluene. The reactor was heated in an oil bath at 70°C and stirred under argon, and to it nickel chloride (26 mg, 0.2 mmole) and zinc dust (2.0 g, 30 mmoles) were added. After 20 hours, an additional portion of nickel chloride (20 mg) was added and the mixture allowed to react for another 24 hours. The solution was filtered to remove inorganic materials and the filtrate mixed with 300 mL of ether. The product was collected as bright-yellow granules by filtration and washed with more ether and dried in a vacuum oven at 70°C overnight. The polymer had an inherent viscosity of 0.37 dL/g, Mw 36,280 Daltons, Mn 20,700 Daltons, polydispersity of 1.75. The chain structure of the polymer is as follows:

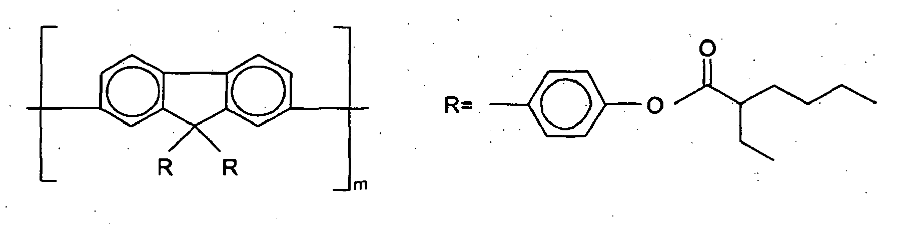

A solution of the resulting polymer in THF showed a broad absorption maximum at 389 nm, molar extinction coefficient of 50,000 and photoluminescent peaks at 417, 439, and 473 nm in decreasing intensity.

### Example 18 - Poly(9,9-di(2-ethylhexyl)fluorene-2,7'-diyl)

A reactor was charged with 2,7-dibromo-9,9-di(2-ethylhexyl)fluorene (2.75 g, 5 mmoles), zinc dust (0.98 g, 15 mmoles), a NiCl₂-bipyridine complex (21.5 mg, 0.075 mmole), and TPP (0.66 g, 2.5 mmoles). The reactor was evacuated and filled with nitrogen several times. N-cyclohexylpyrrolidinone (3 mL) was added and the contents were stirred under nitrogen in a 80°C oil bath. After 22 hours, the oil bath temperature was raised to 90°C and the polymerization was allowed to proceed for a total of 31 hours. The reaction mixture was mixed with chloroform and filtered. The filtrate was concentrated and blended with methanol to give a yellow solid. The solid was dissolved in toluene (15 mL) and percolated through a short silica gel column with cyclohexane. The cyclohexane solution was concentrated and blended with methanol to precipitate the polymer as a yellow solid. Inherent viscosity was 0.15 dL/g. The chain structure of the polymer is as follows:

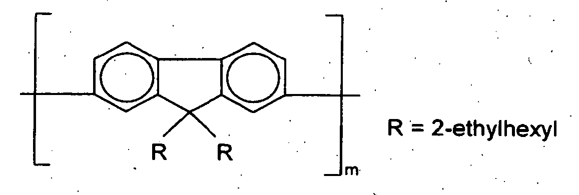

A small piece of the polymer was melted on a microscope slide at 230°C and slowly cooled to room temperature while being examined under cross-polarized light. Birefringence emerged at 175°C. Heating the same film from room temperature revealed a liquid crystal to isotropic transition at 165°C.

### Example 19 - Copolymer

To a reactor was added 2,7-dichloro-9,9-di(2-ethylhexyl)fluorene (0.92 g, 2 mmoles), 2,7-dichloro-9,9-di-n-hexylfluorene (3.2 g, 8 mmoles), TPP (1.31 g, 5.0 mmoles), NiCl₂-bipyridine complex (43 mg, 0.15 mmole) and zinc dust (1.96 g, 30 mmoles). The reactor was evacuated and filled with nitrogen several times and then to it was added DMAc (10 mL). The mixture was stirred at 80°C for 7 hours and at 90°C for 15 hours. The reaction mixture was mixed with chloroform and filtered. The filtrate was concentrated and the copolymer precipitated in acetone and dried in a vacuum oven at 50°C overnight. The chain structure of the copolymer is as follows:

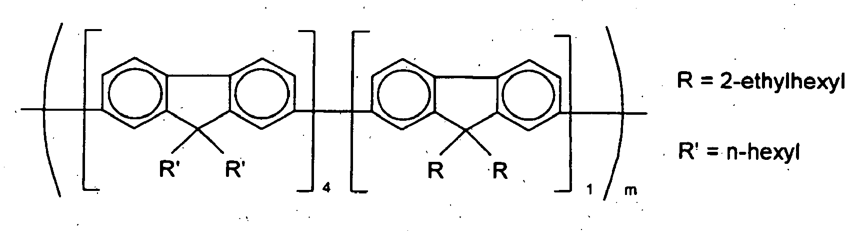

The copolymer had an inherent viscosity of 0.35 dL/g and a Tg at 89°C as measured by DSC.

### Example 20 - Poly(9,9-di(2-methoxycarbonylethyl)-fluorene-2,7'-diyl)

A reactor was charged with TPP (2.0 g, 7.67 mmoles), zinc dust (2.02 g, 30.9 mmoles) and nickel bromide (0.22 g, 1.0 mmole). The reactor was evacuated and filled with nitrogen several times. To the reactor was added dimethylformamide (DMF) (5 mL) and the mixture was stirred at 40°C for 15 minutes. Then a solution of 2,7-dibromo-9,9-di(2-methoxycarbonylethyl)fluorene (4.96 g, 10 mmoles) in DMF (10 mL), previously flushed with nitrogen, was added. The polymerization was allowed to proceed for 48 hours at 80°C. The reaction mixture was dissolved in chloroform and filtered. The chloroform layer was washed with water and dried over anhydrous magnesium sulfate. Upon removal of the solvent, a yellow granular solid was obtained with the following chain structure:

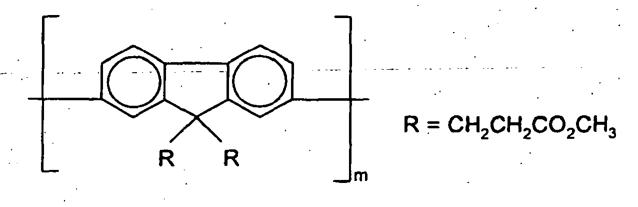

### Example 21 - Benzocyclobutene (BCB)-capped oligo(9,9-di(2-ethylhexyl)fluorene-2,7'-diyl)

A reactor was-charged with 2,7-dibromo-9,9-di(2-ethylhexyl)fluorene (11.0 g, 20 mmoles), zinc dust (3.92 g, 60.0 mmoles), nickel complex (172 mg, 0.6 mmole) and TPP (2.62 g, 10 mmoles). The reactor was evacuated and filled with nitrogen several times. A solution of 4-bromobenzocyclobutene (Br-BCB) (1.10 g, 6 mmoles) in DMF (20 mL), previously flushed with nitrogen, was added to the reactor. The content was stirred at 75°C under nitrogen for 24 hours. The reaction mixture was dissolved in 50 mL of toluene and filtered and the filtrate washed with water. The toluene solution was stirred at room temperature with 5 mL of 70 percent t-butylhydroperoxide overnight. Excess peroxide was decomposed with aqueous sodium hydrogen sulfite and the toluene solution washed with water and evaporated to dryness. The residue was extracted with hexane separating the desired product from triphenylphosphine oxide. The hexane solution was percolated through a silica gel column and evaporated to give 5.1 g of the following resin:

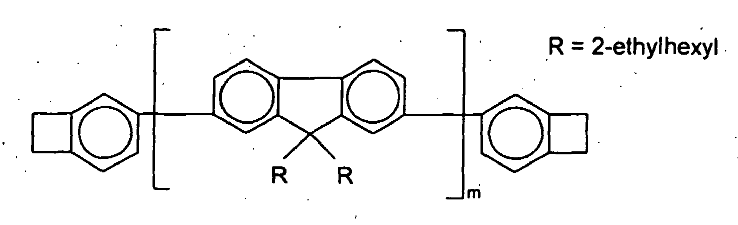

Proton NMR analysis of the product indicated m = 7.4.

A thin film of the material was examined on a hot-stage microscope under cross-polarized light. It was intensely birefringent, indicating presence of liquid crystallinity. Heating to 150°C brought the disappearance of birefringence, which re-emerged upon cooling to room temperature.

### Example 22 - BCB-capped oligo(9,9-di(2-ethylhexyl)-fluorene-2,7'-diyl)

The experiment described in Example 21 was repeated with 5.55 g (10.0 mmoles) of fluorene monomer, 0.54 g (3 mmoles) of Br-BCB, 1.91 g (29.2 mmoles) of zinc dust, 79 mg (0.28 mmole) of nickel complex, 1.31 g (5 mmoles) of TPP and 10 mL of DMAc. The reaction was run at 80°C for 16.5 hours. The product has the same structure as in Example 21 except m = 7.2, as determined by proton NMR. A thin film of the resin was examined on a hot-stage microscope under cross-polarized light. The film was birefringent at room temperature, indicative of liquid crystallinity. Heating the film at 220°C for 30 minutes and 250°C for 1.5 hours gave an insoluble film, indicating that the BCB groups had substantially reacted, giving a crosslinked polymer.

### Example 23 - BCB-capped oligo(9,9-di(2-ethylhexyl)-fluorene-2,7'-diyl)

A 250 mL, three-necked, round-bottomed flask was charged with nickel chloride (0.156 g, 12 mmoles), 2,2'-bipyridine (0.188 g, 12 mmoles), TPP (5.24 g, 20 mmoles) and zinc dust (7.84 g, 120 mmoles) under nitrogen. 40 mL of DMAc was added via a syringe. The gray-colored slurry was stirred and heated at 80°C for 15 minutes when the catalyst mixture turned reddish in color. A mixture of 2,7-dibromo-9,9-di(2-ethylhexyl)fluorene (21.94 g, 40 mmoles), Br-BCB (2.93 g, 16 mmoles), and 30 mL of DMAc was added to the catalyst dropwise over 15 minutes and the reaction mixture was stirred at 80°C overnight.

The reaction mixture was cooled to room temperature, diluted with 200 mL of hexane and filtered through a bed of filter aid deatomaceous earth, and the filtered aid was washed with hexane (3 x 40 mL). To the filtrate was added 10 mL of a 70 percent t-butylhydroperoxide solution and the resulting mixture was stirred at room temperature overnight and filtered through a filter aid. The top hexane layer of the filtrate was recovered, washed with saturated NaCl solution, water and dried over MgSO₄. The hexane solution was concentrated to 50 mL, cooled at 0°C for a few hours to precipitate excess triphenylphosphine oxide, and the filtrate was evaporated to provide a yellow, semi-solid (16 g, 93 percent yield). The material was found to have a structure similar to that of Example 21 but with m = 5.3.

Other properties of the material in the solid state were as follows: liquid crystallinity up to 100°C; strong UV absorption (1 max 365 nm); and photoluminescence at 419 nm with minor peaks at 447, 473, and 511 nm in decreasing intensity. A thin film was cured at 200°C for 1 hour, 225°C for 1 hour and 250°C for 1 hour. The UV absorption of the film was essentially the same before and after cure but the major photoluminescent peak has shifted to 512 nm.

### Example 24 -BCB-capped oligo(9,9-di-n-hexyl)fluorene-2,7'-diyl)

A reactor was charged with 2,7-dichloro-9,9-di-n-hexylfluorene (8.1 g, 20 mmoles) , Br-BCB (1.1 g, 6 mmoles), nickel complex (172 mg, 0.6 mmole), zinc dust (3.92 g, 60 mmoles), TPP (2.62 g, 20 mmoles) and 30 mL of a 1:4 mixture of xylene and DMF. The reaction was stirred under nitrogen at 80°C. After 1.5 hours, an additional 10 mL of solvent mix was added and stirring was continued for 20 hours. The product mixture was dissolved in chloroform (100 mL) and filtered. Chloroform was removed under vacuum and the residue stirred with acetone (600 mL). The acetone-insoluble solid was isolated by filtration and dried in vacuo at 60°C overnight to give 3.89 g of bright-yellow granules which was identified by proton NMR as follows :

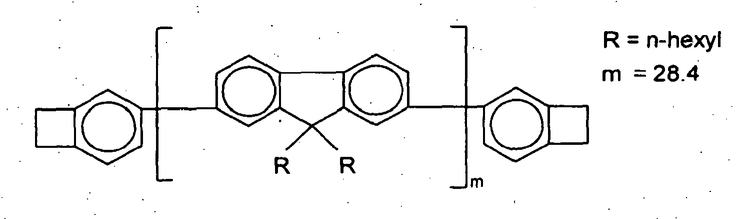

A solid film of this material showed UV absorption at 382 nm and photoluminescent peaks at 427, 440, and 481 nm in decreasing intensity.

The acetone solution was concentrated and mixed with ethanol (300 mL). The precipitated solid was isolated and dried to give 2.22 g of pale-yellow granules which was identified by proton NMR as follows:

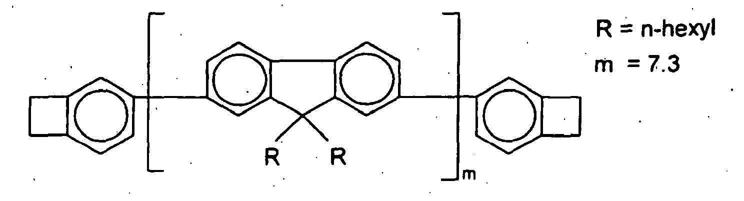

A solid film of this material showed UV absorption at 363 nm and photoluminescent peaks at 421, 442, and 475 nm in decreasing intensity.

Curing the above films as per the procedure of Example 22 did not bring significant shifts in the emission wavelengths. DSC analysis of both materials showed the expected exotherms due to crosslinking reactions of BCB.

### Example 25 - BCB-capped oligo(9,9-di(2-methoxycarbonylethyl)fluorene-2,7'-diyl)

A reactor was charged with 2,7-dibromo-9,9-di(2-methoxycarbonylethyl)fluorene (9.92 g, 20 mmoles), nickel complex (172 mg, 0.6 mmole), zinc dust (2.93 g, 60 mmoles) and TPP

(2.62 g, 10 mmoles). It was evacuated and filled with nitrogen several times. To it was added a solution of Br-BCB (1.46 g, 8 mmoles) in DMAc (30 mL), previously flushed with nitrogen for 45 minutes. The mixture was stirred at 80°C for 20 hours. The reactions product was diluted with chloroform (300 mL) and filtered. The filtrate was concentrated and slurried with ethanol (300 mL) to give a fine, light-yellow solid which was filtered and washed with methanol. The product was dried in vacuum at 50°C overnight. Proton NMR analysis indicated that the structure was as follows:

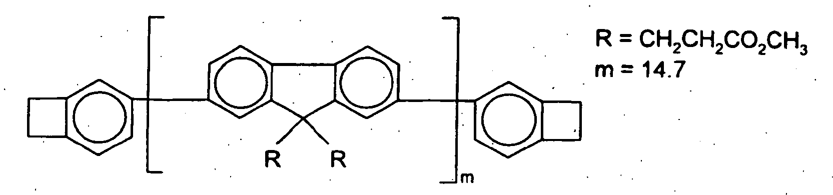

The resin showed multiple transitions during the first DSC scan from 25°C to 300°C. A re-scan showed a Tg of 151°C. A solid film of this material showed UV absorption at 368 nm and photoluminescent peak at 431 and 466 nm of the same intensity, the latter being much broader. Curing the sample according to the procedure of Example 22 caused a shift of the emission peak to 540 nm.

### Example 26 - BCB-capped oligo(9,9-di(2-ethylhexyl)-fluorene-2,7'-diyl)

The experiment described in Example 21 was repeated with 37.9 g (60 mmoles) of 2,7-dibromo-9,9-di(2-ethylhexyl)fluorene, 11.8 g (180 mmoles) of zinc dust, 4.4 g (24 mmoles) of Br-BCB, 7.8 g (30 mmoles) of TPP, 0.52 g (1.8 mmoles) of nickel complex and 90 mL of DMAc. After 23 hours of reaction at 80°C, the content of the reactor was mixed with toluene and stirred with aqueous HCl to digest the excess zinc. The toluene solution was washed with water twice and evaporated to a yellow syrup which was dissolved in hexane (500 mL) and stirred overnight with 50 mL of 70 percent t-butylhydroperoxide. Excess peroxide was destroyed by stirring with an aqueous solution of sodium hydrogen sulfite and the hexane solution washed with water several times. The yellow oil obtained from evaporation of the solvent was passed through a short column (2-inch (5 cm) diameter and 2-inch (5 cm) height) of silica gel. The desired product was eluted with several liters of hexane. The combined hexane solution was again evaporated and the residue dissolved in 80 mL of toluene. The toluene solution was poured as a thin stream into 1.2 L of stirred methanol. The solid cake deposited was collected, dissolved in hexane and the solution dried over anhydrous magnesium sulfate. The solution was evaporated to leave an oil which was further stripped of volatile components at 90°C, 0.5 mm Hg (66 Pa), yielding 14.8 g of BCB-capped oligomer with the following structure:

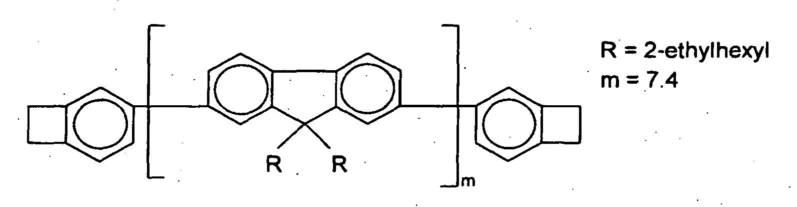

A one-gram sample of the material was placed in a round-bottom flask which was alternately evacuated and filled with argon several times. The flask was then placed in an oil bath heated to 150°C and evacuated to 0.3 mm Hg (40 Pa) for 30 minutes. After filling the flask with argon, the temperature of the bath was raised to 180°C. The sample was heated at this temperature for 24 hours and was allowed to cool under argon. DSC analysis showed that 44 percent of the BCB groups had reacted during the B-staging process. A xylene solution of the B-staged resin was used to spin-coat a quartz disk. The disk was heated in a nitrogen-purged oven from 25°C to 250°C at a ramp rate of 3°C/minute and held at 250°C for 1 hour. This provided a highly photoluminescent (blue), smooth, crosslinked film essentially free of pin-holes and other defects.

### Example 27 - Hydroxyphenyl-capped oligo(9,9-di(2-ethylhexyl)fluorene-2,7'-diyl)

Example 21 was repeated with 19 g (30 mmoles) of 2,7-dibromo-9,9-di(2-ethylhexyl)fluorene, 6 g (90 mmoles) of zinc dust, 3.45 g (12 mmoles) of 4-bromophenyl-t-butyldimethylsilyl ether, 3.9 g (15 mmoles) of TPP, 0.26 g (0.9 mmole) of nickel complex and 50 mL of DMAc. The reaction was allowed to proceed at 80°C for 21 hours. The reaction mixture was diluted with toluene (200 mL) and filtered through a filter aid to remove unreacted zinc. The filtrate was removed of toluene on a rotary evaporator and the residue shaken with water and extracted with hexane. The hexane solution was filtered through a filter aid again and the filtrate stirred with 15 mL of 70 percent t-butylhydroperoxide overnight. Excess peroxide was decomposed with aqueous sodium hydrogen sulfite and the solution washed repeatedly with water. Triphenylphosphine oxide was removed by filtration and the hexane solution was dried over anhydrous magnesium sulfate. Evaporation of hexane and further stripping of volatiles at 0.5 mm Hg (66 Pa), 120°C, 1 hour, gave a semi-solid. This was dissolved in toluene (60 mL) and the toluene solution poured as a thin stream into methanol. The semi-solid precipitated cake was dissolved in hexane. The hexane solution yielded 10.0 g of strongly photoluminescent (blue) semi-solid after drying and stripping. The NMR spectrum of this material is consistent with the expected structure as follows:

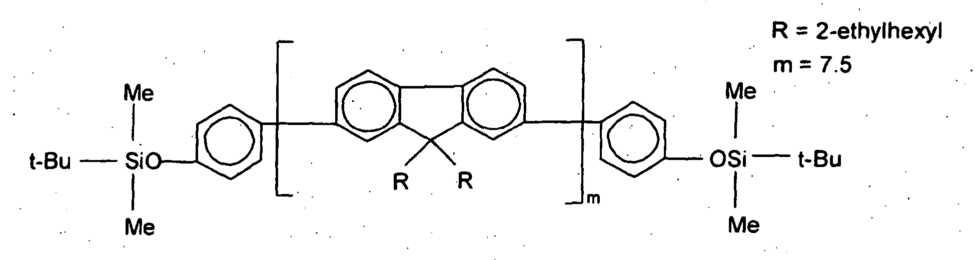

The above material was refluxed with a solution of tetrahydrofuran (100 mL) and concentrated HCl (5 mL) for 6 hours to effect desilylation. The NMR spectrum of the product was consistent with the expected structure as follows.

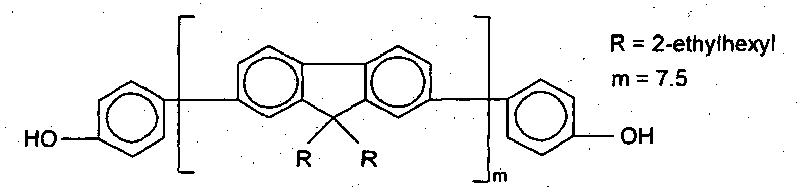

### Example 28 - Cyanatophenyl-capped oligo(9,9-di(2-ethylhexyl)fluorene-2,7'-diyl)

The phenolic material from Example 27 (6.5 g) was dissolved in methylene chloride (75 mL) and mixed with 2.5 g of cyanogen bromide in a reactor. The resulting solution was cooled to -20°C and to it was added a solution of 3 mL triethylamine and 10 mL methylene chloride over a period of 3 minutes. The mixture was stirred at -10°C for 1 hour and was then washed with dilute HCl, water and dried over anhydrous magnesium sulfate. Removal of solvent gave 6.5 g of light-brown oil showing the expected infrared absorption bands (2200 to 2300 cm⁻¹) for cyanate groups and no absorption band for phenolic groups. The oil was further purified by passage through a short silica gel column to give a strongly photoluminescent (blue), yellow semi-solid. The NMR and infrared spectra of the material are consistent with the following structure:

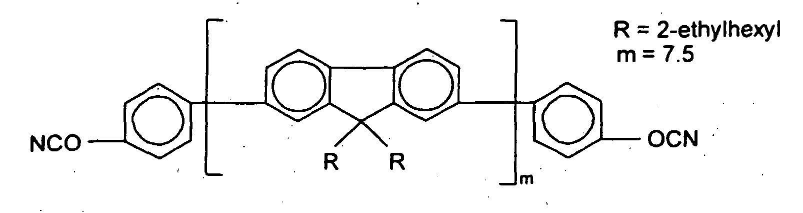

### Example 29 - Polymerization of 2,7-dichloro-9-benzylidenylfluorene

To a dry reactor equipped with a mechanical stirrer, rubber septa, and an inlet connected to a vacuum and nitrogen manifold was added TPP (2.00 g, 8 mmoles), zinc dust (3.02 g, 31 mmoles), and nickel complex (0.26 g, 1 mmole). The reactor was evacuated and purged with nitrogen 7 times and was heated to 40°C. 1-Methyl-2-pyrrolidinone (NMP) (10 mL) was added via a syringe. The reaction mixture was stirred at approximately 250 rpm. After a few minutes, the solution turned to a reddish-brown color. To the reaction mixture was added a solution of 2,7-dichloro-9-benzylidenylfluorene (3.23 g, 10 mmoles) in NMP (7.0 mL). The reaction was stirred at 80°C for 48 hours.

The reaction mixture was poured into acetone (300 mL) to precipitate the polymer. The orange-gray precipitate was collected and washed with acetone (2 x 100 mL). After drying in air, the solid was crushed to a powder and the powder was slowly added to 250 mL of 3N HCl. The mixture was stirred for 3 hours, at which point the precipitate was a bright-orange color. The solid was collected, washed with water (3 x 100 mL) then air-dried overnight. The solid was then dried in a vacuum oven at 60°C overnight to afford 2.46 g (98 percent yield) of an orange powder. Based on the yield and spectral data, the chain structure of the polymer was as follows:

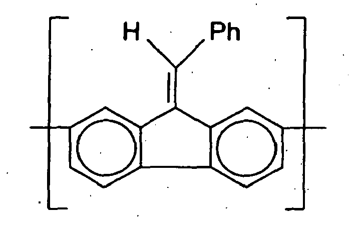

A film of the polymer, cast onto a quartz disk from a chloroform solution, showed a strong UV absorption band centered at 368 nm and a broad photoluminescent band centered at 571 nm.

### Example 30 - Copolymerization (1:1) of 2,7-dichloro-9-benzylidenylfluorene and 2,7-dichloro-9,9-di-n-hexylfluorene

The procedure of Example 29 was repeated with 1.62 g (5 mmoles) of 2,7-dichloro-9-benzylidenylfluorene and 2.02 g (5 mmoles) 2,7-dichloro=9;9-di-n-hexylfluorene in DMAc (15.0 mL). The reaction mixture was stirred at 80°C for 48 hours. The reaction mixture was added to methylene chloride (200 mL) and the resulting solution was filtered through a plug of filter aid to remove the unreacted zinc. The filtered solution, after concentration, was slowly added to acetone (300 mL) to precipitate the product. The bright-yellow precipitate was collected and washed with acetone (3 x 50 mL) and dried to give 1.79 g of 1:1 copolymer whose structure could be alternating as shown below or could consist of blocks of the two monomeric units. A film, cast on a quartz disk from a chloroform solution, showed a strong UV absorption band centered at 370 nm and photoluminescent bands centered at 417 and 551 nm. This copolymer is represented by the following chain structure:

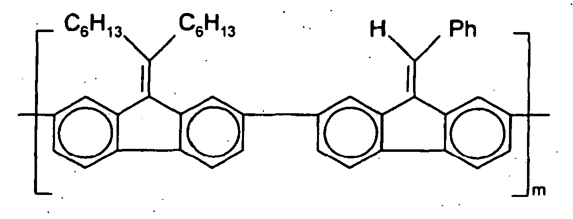

### Example 31 - Copolymerization (1:4) of 2,7-dichloro-9-benzylidenylfluorene and 2,7'-dichloro-9,9-di-n-hexylfluorene

Example 30 was repeated with 0.65 g (2 mmoles) of 2,7-dichloro-9-benzylidenylfluorene and 3.23 g (8 mmoles) of 2,7'-dichloro-9,9-di-n-hexylfluorene in NMP (15 mL). The reaction mixture was stirred at 80°C for 48 hours and was then added to methylene chloride (200 mL). The methylene chloride solution was filtered through a plug of filter aid to remove the zinc. The filtered solution, after concentration, was slowly added to acetone (200 mL) to give 1.0 g of yellow polymer. The acetone solution was concentrated and slowly added to ethanol (200 mL) to precipitate an additional portion of polymeric product (1.3 g). Based on the yield and spectral data, the chain structure of the polymer is as follows:

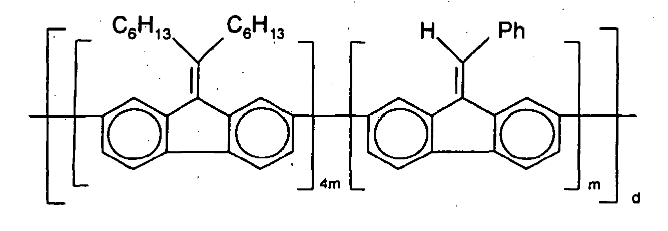

A film, cast on a quartz disk from a chloroform solution, showed a strong UV absorption band centered at 370 nm and photoluminescent bands centered at 470 and 522 nm.

### Example 32 - Polymerization of 2,7-dichloro-9-(3,5,5-trimethylhexylidenyl)fluorene

The procedure of Example 29 was repeated with 4.5 g (10 mmoles) of 2,7-dichloro-9-(3,5,5-trimethylhexylidenyl)fluorene in DMF (15 mL). The reaction mixture was stirred at 80°C for 28 hours. The reaction mixture was added to acetone (200 mL) to precipitate the product. The precipitate was collected, air-dried, crushed to a powder, then added to an aqueous solution of 3N HCl (300 mL). The mixture was stirred for 3 hours to dissolve the zinc metal. The bright, yellow-orange precipitate was collected, washed with methanol (3 x 100 mL), acetone (3 x 100 mL) and dried to give 2.5 g of product. The product was soluble in hot 1,2-dichlorobenzene. The melting point was 306°C.

### Example 33 - Polymerization of 2,7-dichloro-9-(5-norbornenylid-2-ene)fluorene

The procedure of Example 29 was repeated with 4.3 g (10 mmoles) of 2,7-dichloro-9-(5-norbornenylid-2-ene)-fluorene in DMF (20 mL). The reaction mixture was stirred at 80°C for 26 hours. The reaction mixture was added to acetone (200 mL) to precipitate the product. The precipitate was collected, air dried, crushed to a powder, then added to an aqueous solution of 3N HCl (300 mL). The mixture was stirred for 3 hours to dissolve the zinc metal. The precipitate was collected, washed with methanol (3 x 100 mL), acetone (3 x 100 mL) and dried to give 3.0 g of product.

## Claims

1. A compound of the formula: 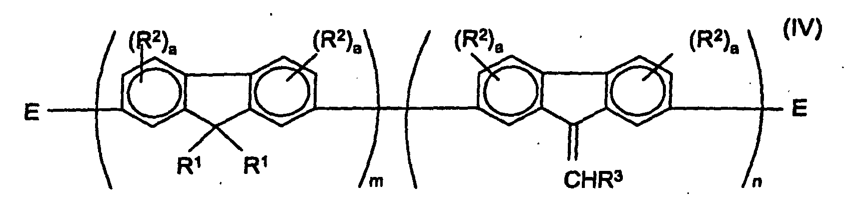 wherein:
E is independently in each occurrence halogen, aryl or aryl substituted with a reactive group capable of undergoing chain extension or crosslinking or a trialkylsiloxy moiety;
R¹ is independently in each occurrence C₁₋₂₀ hydrocarbyl or C₁₋₂₀ hydrocarbyl containing one or more heteroatoms selected from S, N, O, P and Si; C₄₋₁₆ hydrocarbyl carbonyloxy, or (C₉₋₁₆ aryl)trialkylsiloxy, or both R¹ may form, with the 9-carbon of the fluorene ring, a C₅₋₁₀ aliphatic ring structure or a C₄₋₂₀ ring structure containing one or more heteroatoms selected from S, N and O;
R² is independently in each occurrence. C₁₋₂₀ hydrocarbyl-, C₁₋₂₀ hydrocarbyloxy, C₁₋₂₀ thioether, C₁₋₂₀ hydrocarbyloxycarbonyl, C₁₋₂₀ hydrocarbylcarbonyloxy, cyano, thiocyano, C₆₋₁₂ thioaryl, C₁₋₂₀ alkylthio or hydroxy;
R³ is independently in each occurrence C₁₋₂₀ hydrocarbyl or C₁₋₂₀ hydrocarbyl substituted with di(C₁₋₂₀ alkyl)amino, C₁₋₂₀ hydrocarbyloxy, tri (C₁₋₁₀ alkyl) siloxy or C₁₋₂₀ hydrocarbyl;
a is independently in each occurrence 0 or 1; and
m and n are 0 or non-negative numbers and the sum of n and m is at least 10 and
wherein the polymers have a polydispersity of less than 5.

2. A compound of the formula: 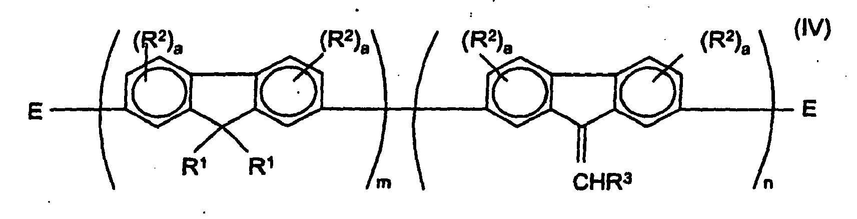 wherein:
E is a phenolic, a cyanato-substituted phenyl or a benzocyclobutene moiety,
R¹ is independently in each occurrence C₁₋₂₀ hydrocarbyl or C₁₋₂₀ hydrocarbyl containing one or more heteroatoms selected from S, N, O, P and Si; C₄₋₁₆ hydrocarbyl carbonyloxy, or (C₉₋₁₆ aryl) trialkylsiloxy, or both R¹ may form, with the 9-carbon of the fluorene ring, a C₅₋₂₀ ring structure or a C₄₋₂₀ ring structure containing one or more heteroatoms selected from S, N and O;
R² is independently in each occurrence C₁₋₂₀ hydrocarbyl, C₁₋₂₀ hydrocarbyloxy, C₁₋₂₀ thioether, C₁₋₂₀ hydrocarbyloxycarbonyl, C₁₋₂₀ hydrocarbylcarbonyloxy, cyano, thiocyano, C₆₋₁₂ thioaryl, C₁₋₂₀ alkylthio or hydroxy;
R³ is independently in each occurrence C₁₋₂₀ hydrocarbyl or C₁₋₂₀ hydrocarbyl substituted with di C₁₋₂₀ alkyl) amino, C₁₋₂₀ hydrocarbyloxy, tri(C₁₋₂₀ alkyl) siloxy or C₁₋₂₀ hydrocarbyl;
a is independently in each occurrence 0 or 1; and
m and n are 0 or non-negative numbers and the sum of n and m is greater than 1 and
wherein the polymers have a polydispersity of less than 5.

3. A compound of Claim 2, wherein the sum of n and m is at least 10.

4. A compound of Claim 1 or Claim 3, wherein m is a number of 1 or greater and n is 0 or a number of 1 or greater (subject to the limitation on m and n in the relevant claim).

5. A compound of any one of Claims 2 to 4, wherein m is a number of 1 to 100 and n is 0 or a number of 1 to 100 (subject to the limitation on m and n in the relevant claim).

6. A compound of any one of Claims 1, 4 and 5, wherein E is independently in each occurrence an aryl; an aryl substituted with a hydroxy, a glycidyl ether, acrylate ester, methacrylate ester, ethenyl, ethynyl, maleimide, nadimide, trialkylsiloxy, trifluorovinyl ether moiety; or a benzocyclobutene.

7. A compound of Claim 1, wherein E is halogen, cyanato, aryl or aryl substituted with a reactive group capable of undergoing chain extension or crosslinking or a trialkylsiloxy moiety.

8. A compound of Claim 7, wherein E is aryl or aryl substituted with a reactive group capable of undergoing chain extension or crosslinking or a trialkylsiloxy.

9. A compound of Claim 8, wherein E is an aryl - moiety having an acrylate or methacrylate ester reactive group corresponding to the formula 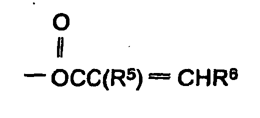 or an ethenyl moiety corresponding to the formula
-C(R⁷)= C(R⁷)H
wherein:
R⁵ is independently in each occurrence hydrogen or C₁₋₄ alkyl;
R⁶ is independently in each occurrence hydrogen, C₁₋₂₀ hydrocarbyl; or C₁₋₂₀ hydrocarbyloxy and
R⁷ is independently in each occurrence hydrogen, C₁₋₂₀ hydrocarbyl or C₃₋₂₀ hydrocarbyloxy.

10. A compound as claimed in Claim 2, wherein E is a benzocyclobutene moiety corresponding to the formula 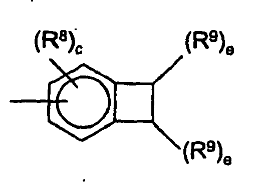 wherein
R⁸ is independently in each occurrence C₁₋₂₀ alkyl, C₁₋₂₀ alkoxy, C₁₋₂₀ alkylthio, C₆₋₂₀ aryl, C₆₋₂₀ aryloxy, C₆₋₂₀ arylthio, C₇₋₂₀ aralkoxy, C₇₋₂₀ alkaryloxy, C₇₋₂₀ alkarylthio, C₇₋₂₀ aralkyl, C₇₋₂₀ aralkylthio, cyano, carboxylate, C₁₋₂₀ hydrocarbylcarbonyloxy, C₁₋₂₀ hydrocarbylsulfinyl, C₁₋₂₀ hydrocarbylsulfonyl, amino or C₁₋₂₀ dialkylamino;
R⁹ is independently in each occurrence cyano, carboxylate, C₁₋₂₀ hydrocarbylcarbonyloxy, nitro, halo, C₁₋₂₀ hydrocarbylsulfonyl, C₁₋₂₀ hydrocarbylsulfinyl, C₁₋₂₀ alkyl, amido, or C₁₋₂₀ hydrocarbyloxy;
c is independently in each occurrence 0 or an integer of 1 to 3; and
e is independently in each occurrence 0, or 1.

11. A compound according to any one of Claims 1 to 10 which is an oligomer or polymer according to Formula IX, 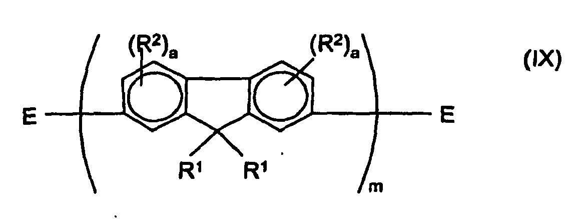 wherein:
R¹, R², and a are as defined in Claim 1;
E is as defined in any one of Claims 4 to 9; and
m is greater than 1 for compounds of Claim 2 and otherwise greater than 10.

12. A compound according to any one of Claims 1 to 10 which is an oligomer or polymer according to Formula X, 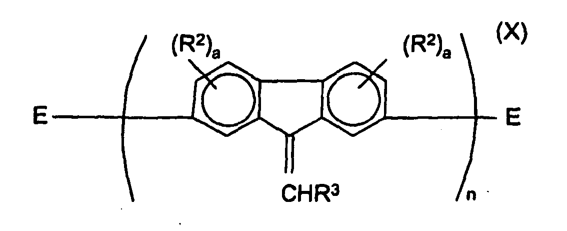 wherein:
R², R³, and a are as defined in Claim 1;
E is as defined in any one of Claims 2 and 6 to 10; and
n is greater than 1 for compounds of Claim 2 and otherwise greater than 10.

13. A compound according to Claim 12 which is an oligomer or polymer according to Formula XI, 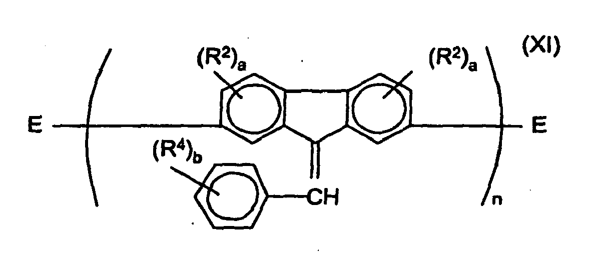 R² and a are as defined in Claim 1;
R⁴ is independently in each occurrence di (C₁₋₂₀ alkyl) amino, C₁₋₂₀ hydrocarbyloxy, tri (C₁₋₁₀ alkyl) siloxy or C₁₋₂₀ hydrocarbyl;
E is as defined in any one of Claims 2 and 6 to 10;
b is independently 0 or a number of from 1 to 3; and
n is greater than 1 for compounds of Claim 2 and otherwise greater than 10.

14. A compound according to any one of Claims 1 to 10 which is an oligomer or polymer according to Formula V, 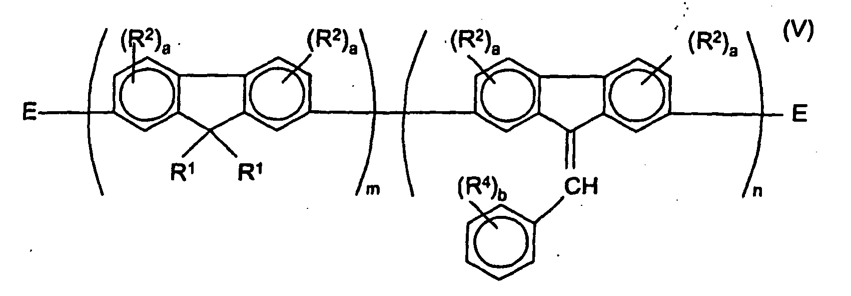 wherein:
R¹, R², and a are as defined in Claim 1;
E is as defined in any one of Claims 2 and 6 to 10;
R⁴ is independently in each occurrence di(C₁₋₂₀alkyl)amino, C₁₋₂₀ hydrocarbyloxy, tri(C₁₋₁₀alkyl)siloxy or C₁₋₂₀ hydrocarbyl;
m and n are each at least 1; and
b is independently in each occurrence 0 or a number of from 1 to 3.

15. A compound according to any one of Claims 1 to 11 and 14, wherein R¹ is C₁₋₁₂ alkyl, C₆₋₁₀ aryl or alkyl-substituted aryl, C₄₋₁₆ hydrocarbyl carboxylate or (C₉₋₁₆ aryl) trialkylsiloxy.

16. A compound according to any one of Claims 1 to 11 and 14, wherein both R¹ form a C₅₋₂₀ straight- or branched-ring structure (subject to being a C₅₋₁₀ aliphatic ring when dependent directly or indirectly on Claim 1) or a C₄₋₂₀ straight- or branched-chain ring structure containing one or more heteroatoms selected from S, N and O.

17. A compound according to Claim 16, wherein both R¹ form a C₅₋₁₀ aliphatic ring or a C₄₋₁₀ aliphatic ring containing one or more heteroatoms selected from S and O.

18. A compound according to Claim 17, wherein both R¹ form a C₅₋₁₀ cycloalkyl or C₄₋₁₀ cycloalkyl containing oxygen .

19. A compound according to any one of the preceding claims, wherein R² is C₁₋₁₂ alkyl, C₆₋₁₀ aryl or alkyl-substituted aryl, C₁₋₁₂ alkoxy, C₁₋₁₂ alkoxycarbonyl, C₆₋₁₀ aryloxycarbonyl or alkyl-substituted aryloxycarbonyl, C₁₋₁₂ alkylcarbonyloxy, C₆₋₁₀ arylcarbonyloxy or alkyl-substituted arylcarbonyloxy, C₆₋₁₀ aryloxy or alkyl-substituted aryloxy, cyano, thiocyano, C₆₋₁₂ thioaryl, C₁₋₂₀ alkylthio, or hydroxy.

20. A substituted fluorene compound according to Claim 19, wherein R² is C₁₋₄ alkoxy, phenoxy, C₁₋₄ alkyl, phenyl or cyano.

21. A compound according to any one of Claims 1 to 12, 19, and 20, wherein R³ is a hydrocarbyl selected from C₁₋₂₀ straight- or branched-chain aliphatics, C₃₋₂₀ straight- or branched-chain aliphatics containing one or more cycloaliphatic rings, C₆₋₂₀ aryls, and C₇₋₂₀ alkyl-substituted aryls wherein the hydrocarbyl optionally is substituted with or more substituents selected from di(C₁₋₂₀ alkyl) amino, C₁₋₂₀ hydrocarbyloxy, C₁₋₂₀ hydrocarbyl and tri(C₁₋₁₀ alkyl)-siloxy.

22. A compound according to Claim 21, wherein R³ is a C₁₋₂₀ straight- or branched-chain aliphatic, a C₃₋₂₀ straight- or branched-chain aliphatic containing one or more cycloaliphatic rings or a phenyl moiety and such moiety may optionally be substituted with a di(C₁₋₂₀ alkyl) amino, C₁₋₂₀ hydrocarbyl, tri (C₁₋₁₀ alkyl) siloxy or C₁₋₂₀ hydrocarbyloxy moiety.

23. A compound according to Claim 22, wherein R³ is a C₃₋₁₀ aliphatic, a C₃₋₁₀ aliphatic containing one or more cycloaliphatic moieties, phenyl or phenyl substituted with di(C₁₋₁₂ alkyl)amino, C₁₋₁₀ alkoxy, C₆₋₁₀ aryloxy or alkyl-substituted aryloxy, C₁₋₁₀ alkyl or C₆₋₁₀ aryl or alkyl-substituted aryl or tri (C₁₋₄ alkyl)siloxy.

24. A compound according to Claim 23, wherein R³ is phenyl or phenyl substituted with di (C₁₋₆ alkyl) amino, C₁₋₁₀ alkoxy or C₁₋₁₀ alkyl.

25. A compound according to any one of Claims 13 to 20, wherein R⁴ is di(C₁₋₁₂ alkyl) amino, C₁₋₁₀ alkoxy, C₆₋₁₀ aryloxy or alkyl-substituted aryloxy, tri(C₁₋₄ alkyl)siloxy, C₁₋₂₀ alkyl, or C₆₋₁₀ aryl or alkyl-substituted aryl.

26. A compound according to Claim 25, wherein R⁴ is di(C₁₋₆ alkyl) amino, C₁₋₁₀ alkoxy or C₁₋₁₀ alkyl.

27. A compound according to Claim 25, wherein R⁴ is C₁₋₁₀ alkyl, C₆₋₁₀ aryl or C₇₋₁₀ alkyl aryl.

28. A compound according to any one of Claims 13 to 20, and 25 to 27, wherein b is 1.

29. A compound according to any one of Claims 1 to 28 which has a weight average molecular weight of 10,000 or greater and a polydispersity of 3.0 or less.

30. A substituted fluorene compound according to any one of Claims 1 to 29, wherein a is 0.

31. A process for preparing a 9-substituted fluorene oligomer or polymer as defined in Claim 1 or Claim 2 which comprises contacting one or more 2,7-dihalo-9-substituted fluorenes with a haloaromatic compound in the presence of a catalyst for the de-halogen-coupling of aryl halides.

32. A process for preparing a 9-substituted fluorene oligomer or polymer which comprises contacting one or more 2,7-dihalo-9-substituted fluorenes and optionally one or more haloaromatic compounds, in the presence of a catalytic amount of a divalent nickel salt, at least a stoichiometric amount of zinc powder, a trihydrocarbylphosphine in a polar solvent and an optional co-solvent comprising an aromatic hydrocarbon or ether under reaction conditions sufficient to form the corresponding 9-substituted fluorene oligomer or polymer.

33. A process according to Claim 32, wherein the 2,7-dihalo-9-substituted fluorene corresponds to the following formula, 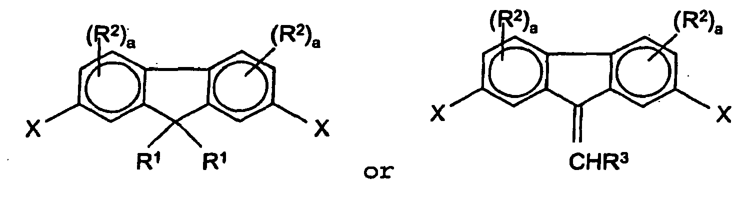 wherein:
R¹ is independently in each occurrence C₁₋₂₀ hydrocarbyl or C₁₋₂₀ hydrocarbyl containing one or more heteroatoms selected from S, N, O, P and Si; C₄₋₁₆ hydrocarbyl carbonyloxy, or (C₉₋₁₆ aryl) trialkylsiloxy, or both R¹ may form, with the 9-carbon of the fluorene ring, a C₅₋₂₀ ring structure or a C₄₋₂₀ ring structure containing one or more heteroatoms selected from S, N and O;
R² is independently in each occurrence C₁₋₂₀ hydrocarbyl, C₁₋₂₀ hydrocarbyloxy, C₁₋₂₀ thioether, C₁₋₂₀ hydrocarbyloxycarbonyl, C₁₋₂₀ hydrocarbylcarbonyloxy, cyano, thiocyano, C₆₋₁₂ thioaryl, C₁₋₂₀ alkylthio or hydroxy;
R³ is independently in each occurrence C₁₋₂₀ hydrocarbyl or C₁₋₂₀ hydrocarbyl substituted with di (C₁₋₂₀ alkyl) amino, C₁₋₂₀ hydrocarbyloxy, tri(C₁₋₁₀ alkyl) siloxy or C₁₋₂₀ hydrocarbyl;
a is independently in each occurrence 0 or 1; and
m and n are 0 or non-negative numbers and the sum of n and m is at least 1; and
X is a halogen moiety.

34. A process of Claim 33, wherein the 2,7-dihalo-9-substituted fluorene corresponds to Formula I: 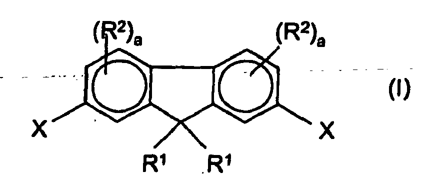 wherein:
R¹, R², and a are as defined in Claim 33 and
X is a halogen moiety.

35. A process according to Claim 34, wherein R¹ is C₁₋₁₂ alkyl, C₆₋₁₀ aryl or alkyl-substituted aryl, C₄₋₁₆ hydrocarbyl carboxylate or (C₉₋₁₆ aryl) trialkylsiloxy.

36. A process according to 35 wherein both R¹ form a C₅₋₂₀ straight- or branched-ring structure or a C₄₋₂₀ straight- or branched-chain ring structure containing one or more heteroatoms selected from S, N and O.

37. A process according to Claim 36, wherein both R¹ form a C₅₋₁₀ aliphatic ring or a C₄₋₁₀ aliphatic ring containing one or more heteroatoms selected from S and O.

38. A process according to Claim 37, wherein both R¹ form a C₅₋₁₀ cycloalkyl or C₄₋₁₀ cycloalkyl containing oxygen.

39. A process according to any one of Claims 37 to 38, wherein R² is C₁₋₁₂ alkyl, C₆₋₁₀ aryl or alkyl-substituted aryl, C₁₋₁₂ alkoxy, C₁₋₁₂ alkoxycarbonyl, C₅₋₁₀ aryloxycarbonyl or alkyl-substituted aryloxycarbonyl, C₁₋₁₂ alkylcarbonyloxy, C₆₋₁₀ arylcarbonyloxy or alkyl-substituted arylcarbonyloxy, C₆₋₁₀ aryloxy, or alkyl-substituted aryloxy, cyano, thiocyano, C₆₋₁₂ thioaryl, C₁₋₂₀ alkylthio, or hydroxy.

40. A process according to Claim 39, wherein R² is C₁₋₄ alkoxy, phenoxy, C₁₋₄ alkyl, phenyl or cyano.

41. A process according to Claim 33, wherein R³ is a hydrocarbyl selected from C₁₋₂₀ straight- or branched-chain aliphatics, C₃₋₂₀ straight- or branched-chain aliphatics containing one or more cycloaliphatic rings, C₆₋₂₀ aryls, and C₇₋₂₀ alkyl-substituted aryls wherein the hydrocarbyl optionally is substituted with or more substituents selected from di (C₁₋₂₀ alkyl)amino, C₁₋₂₀ hydrocarbyloxy, C₁₋₂₀ hydrocarbyl and tri (C₁₋₁₀ alkyl) siloxy.

42. A process according to Claim 41, wherein R³ is a C₁₋₂₀ straight- or branched-chain aliphatic, a C₃₋₂₀ straight- or branched-chain aliphatic containing one or more cycloaliphatic rings or a phenyl moiety and such moiety may optionally be substituted with a di (C₁₋₂₀ alkyl) amino, C₁₋₂₀ hydrocarbyl, tri (C₁₋₁₀ alkyl) siloxy or C₁₋₂₀ hydrocarbyloxy moiety.

43. A process according to Claim 42, wherein R³ is a C₃₋₁₀ aliphatic, a C₃₋₁₀ aliphatic containing one or more cycloaliphatic moieties, phenyl or phenyl substituted with di(C₁₋₂₀ alkyl) amino, C₁₋₂₀ alkoxy, C₆₋₁₀ aryloxy or alkyl-substituted aryloxy, C₁₋₁₀ alkyl or C₆₋₁₀ aryl or alkyl-substituted aryl or tri (C₁₋₄ alkyl)siloxy.

44. A process according to Claim 43, wherein R³ is phenyl or phenyl substituted with di (C₁₋₆ alkyl)amino, C₁₋₁₀ alkoxy or C₁₋₁₀ alkyl.

45. A process according to Claim 33, wherein the 2,7-dihalo-9-substituted fluorene corresponds to Formula III, 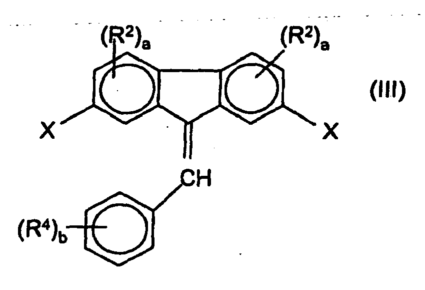 wherein:
R² and a are as defined in Claim 33;
R⁴ is di (C₁₋₁₂ alkyl) amino, C₁₋₁₀ alkoxy, C₁₋₁₀ aryloxy or alkyl-substituted aryloxy, tri(C₁₋₄alkyl)siloxy, C₁₋₁₀ alkyl, or C₆₋₁₀ aryl or alkyl-substituted aryl and
b is 0 or a number of from 1 to 3.

46. A process according to claim 45, wherein R⁴ is di (C₁₋₆ alkyl) amino, C₁₋₁₀ alkoxy or C₁₋₁₀ alkyl.

47. A process according to Claim 45, or Claim 46, wherein b is 1.

48. A process of any one of Claims 31 to 47, wherein the haloaromatic compound is of the Formula X-Ar wherein X is halogen and Ar independently in each occurrence an aryl or aryl substituted with a moiety capable of cross-linking or chain extension or a trialkylsiloxy moiety.

49. A process of Claim 48, wherein Ar is aryl or aryl substituted with a hydroxy, a glycidyl ether, acrylate ester, methacrylate ester, ethenyl, ethynyl, maleimide, nadimide or trifluorovinyl ether moiety or a benzocyclobutene.

50. A process of Claim 49, wherein Ar is an aryl group substituted with an acrylate or methacrylate ester corresponding to the formula 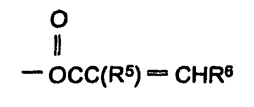 or an ethenyl moiety corresponding to the formula
-C(R⁷)=C(R⁷)H
wherein:
R⁵ is independently in each occurrence hydrogen or C₁₋₄ alkyl;
R⁶ is independently in each occurrence hydrogen, C₁₋₂₀ hydrocarbyl or C₁₋₂₀ hydrocarbyloxy;
R⁷ is independently in each occurrence hydrogen, C₁₋₂₀ hydrocarbyl or C₁₋₂₀ hydrocarbyloxy.

51. A process of Claim 50, wherein Ar is an aryl group substituted by a benzocyclobutene corresponding to the formula, 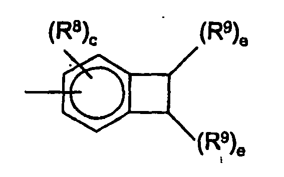 wherein
R⁸ is independently in each occurrence C₁₋₂₀ alkyl, C₁₋₂₀ alkoxy, C₁₋₂₀ alkylthio, C₆₋₂₀ aryl, C₆₋₂₀ aryloxy, C₆₋₂₀ arylthio, C₇₋₂₀ aralkoxy, C₇₋₂₀ alkaryloxy, C₇₋₂₀ alkarylthio, C₇₋₂₀ aralkyl, C₇₋₂₀ aralkylthio, cyano, carboxylate, C₁₋₂₀ hydrocarbylcarbonyloxy, C₁₋₂₀ hydrocarbylsulfonyl, hydrocarbylsulfinyl, amino or C₁₋₂₀ dialkylamino;
R⁹ is independently in each occurrence cyano, carboxylate, C₁₋₂₀-hydrocarbylcarbonyloxy, nitro, halo, C₁₋₂₀ hydrocarbylsulfonyl, C₁₋₂₀ hydrocarbylsulfinyl, C₁₋₂₀ alkyl, amido or C₁₋₂₀ hydrocarbyloxy;
b is independently in each occurrence 0 or an integer of 1 to 3;
c is independently in each occurrence 0 or an integer of 1 to 3; and
e is independently in each occurrence 0 or 1.

52. A process of any one of Claims 31 to 51, wherein the mole ratio of 2,7-dihalo-9-substituted fluorene to haloaromatic compound is from 0.5:1 to 50:1.

53. A process of Claim 52, wherein the process is conducted in the presence of a divalent nickel salt present in an amount of from 0.01 to 20 mole percent based on the amount of haloaromatic compound and 2,7-dihalofluorene, zinc powder present in an amount of 100 mole percent to 300 mole percent based on the haloaromatic compound and 2,7-dihalofluorene, triarylphosphine present in an amount of from 10 to 50 mole percent based on the haloaromatic compound and 2,7-dihalofluorene and a compound capable of accelerating the reaction present in an amount from 100 to 150 mole percent based on the divalent salt.

54. A film of an oligomer or polymer as defined in any one of Claims 1 to 30.

55. A coating of a polymer as defined in any one of Claims 1 to 30.

56. A polymeric light-emitting diode comprising a film or coating as defined in Claim 54 or Claim 55.

## Patentansprüche

1. Verbindung der Formel: 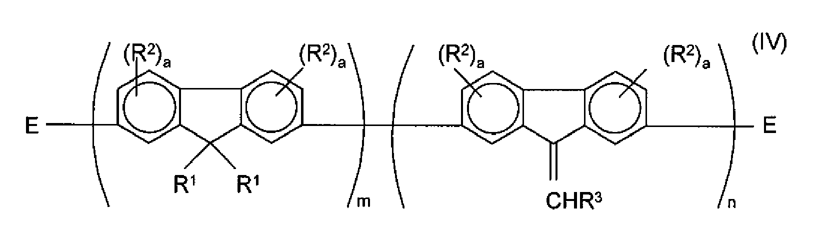 worin:
E unabhängig voneinander bei jedem Auftreten Halogen, Aryl oder Aryl ist, das mit einer reaktiven Gruppe substituiert ist, die fähig ist, Kettenverlängerung oder Vernetzung zu durchlaufen, oder eine Trialkylsiloxyeinheit ist,
R¹ unabhängig voneinander bei jedem Auftreten C₁₋₂₀-Hydrocarbyl oder C₁₋₂₀-Hydrocarbyl mit einem oder mehreren Heteroatomen ausgewählt aus S, N, O, P und Si; C₄₋₁₆-Hydrocarbylcarbonyloxy oder (C₉₋₁₆-Aryl)trialkylsiloxy ist oder beide R¹ mit dem 9-Kohlenstoff des Fluorenrings eine aliphatische C₅₋₁₀-Ringstruktur oder eine C₄₋₂₀-Ringstruktur bilden können, die eine oder mehrere Heteroatome ausgewählt aus S, N und O enthält,
R² unabhängig voneinander bei jedem Auftreten C₁₋₂₀-Hydrocarbyl, C₁₋₂₀-Hydrocarbyloxy, C₁₋₂₀-Thioether, C₁₋₂₀-Hydrocarbyloxycarbonyl, C₁₋₂₀-Hydrocarbylcarbonyloxy, Cyano, Thiocyano, C₆₋₁₂-Thioaryl, C₁₋₂₀-Alkylthio oder Hydroxy ist,
R³ unabhängig voneinander bei jedem Auftreten C₁₋₂₀-Hydrocarbyl oder C₁₋₂₀-Hydrocarbyl, substituiert mit Di(C₁₋₂₀-alkyl)amino, C₁₋₂₀-Hydrocarbyloxy, Tri(C₁₋₁₀-alkyl)siloxy oder C₁₋₂₀-Hydrocarbyl, ist,
a unabhängig voneinander bei jedem Auftreten 0 oder 1 ist und
m und n 0 oder nicht-negative Zahlen sind und die Summe von n und m mindestens 10 beträgt,
und wobei die Polymere eine Polydispersität von weniger als 5 aufweisen.

2. Verbindung der Formel: 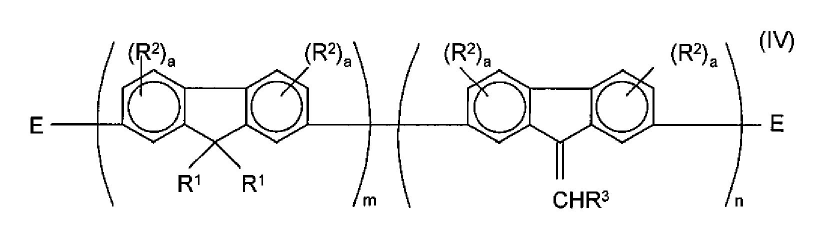 worin:
E eine phenolische, a cyanatosubstituierte Phenyl- oder eine Benzocyclobuten-Einheit ist,
R¹ unabhängig voneinander bei jedem Auftreten C₁₋₂₀-Hydrocarbyl oder C₁₋₂₀-Hydrocarbyl mit einem oder mehreren Heteroatomen ausgewählt aus S, N, O, P und Si; C₄₋₁₆-Hydrocarbylcarbonyloxy oder (C₉₋₁₆-Aryl)trialkylsiloxy ist oder beide R¹ mit dem 9-Kohlenstoff des Fluorenrings eine C₅₋₂₀-Ringstruktur oder eine C₄₋₂₀-Ringstruktur bilden können, die ein oder mehrere Heteroatome ausgewählt aus S, N und O enthält, R² unabhängig voneinander bei jedem Auftreten C₁₋₂₀-Hydrocarbyl, C₁₋₂₀-Hydrocarbyloxy, C₁₋₂₀-Thioether, C₁₋₂₀-Hydrocarbyloxycarbonyl, C₁₋₂₀-Hydrocarbylcarbonyloxy, Cyano, Thiocyano, C₆₋₁₂-Thioaryl, C₁₋₂₀-Alkylthio oder Hydroxy ist,
R³ unabhängig voneinander bei jedem Auftreten C₁₋₂₀-Hydrocarbyl oder C₁₋₂₀-Hydrocarbyl, substituiert mit Di(C₁₋₂₀-alkyl)amino,
C₁₋₂₀-Hydrocarbyloxy, Tri(C₁₋₁₀-alkyl)siloxy oder C₁₋₂₀-Hydrocarbyl, ist,
a unabhängig voneinander bei jedem Auftreten 0 oder 1 ist und
m und n 0 oder nicht-negative Zahlen sind und die Summe von n und m größer als 1 ist,
und wobei die Polymere eine Polydispersität von weniger als 5 aufweisen.

3. Verbindung nach Anspruch 2, worin die Summe von n und m mindestens 10 ist.

4. Verbindung nach Anspruch 1 oder Anspruch 3, worin m eine Zahl von 1 oder größer ist und n gleich 0 oder eine Zahl von 1 oder größer ist (vorbehaltlich der Beschränkung von m und n in dem entsprechenden Anspruch).

5. Verbindung nach einem der Ansprüche 2 bis 4, worin m eine Zahl von 1 bis 100 ist und n gleich 0 oder eine Zahl von 1 bis 100 ist (vorbehaltlich der Beschränkung von m und n in dem entsprechenden Anspruch).

6. Verbindung nach einem der Ansprüche 1, 4 oder 5, worin E unabhängig voneinander bei jedem Auftreten ein Aryl; ein Aryl, das mit einer Hydroxy-, einer Glycidylether-, Acrylatester-, Methacrylatester-, Ethenyl-, Ethinyl-, Maleimid-, Nadimid-, Trialkylsiloxy-, Trifluorvinylethereinheit substituiert ist, oder ein Benzocyclobuten ist.

7. Verbindung nach Anspruch 1, worin E Halogen, Cyanato, Aryl oder Aryl ist, das mit einer reaktiven Gruppe substituiert ist, die fähig ist, Kettenverlängerung oder Vernetzung zu durchlaufen, oder eine Trialkylsiloxyeinheit ist.

8. Verbindung nach Anspruch 7, worin E Aryl oder Aryl ist, das mit einer reaktiven Gruppe substituiert ist, die fähig ist, Kettenverlängerung oder Vernetzung zu durchlaufen, oder Trialkylsiloxy ist.

9. Verbindung nach Anspruch 8, worin E eine Aryleinheit mit einer reaktiven Acrylat- oder Methacrylatestergruppe entsprechend der Formel 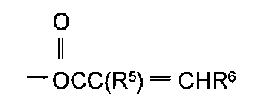 oder einer Ethenyleinheit entsprechend der Formel
-C(R⁷)=C(R⁷)H
ist, worin:
R⁵ unabhängig voneinander bei jedem Auftreten Wasserstoff oder C₁₋₄-Alkyl ist,
R⁶ unabhängig voneinander bei jedem Auftreten Wasserstoff, C₁₋₂₀-Hydrocarbyl oder C₁₋₂₀-Hydrocarbyloxy ist und
R⁷ unabhängig voneinander bei jedem Auftreten Wasserstoff, C₁₋₂₀-Hydrocarbyl oder C₁₋₂₀-Hydrocarbyloxy ist.

10. Verbindung nach Anspruch 2, worin E eine Benzocyclobuteneinheit entsprechend der Formel 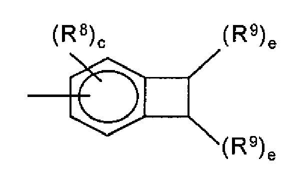 ist, worin
R⁸ unabhängig voneinander bei jedem Auftreten C₁₋₂₀-Alkyl, C₁₋₂₀-Alkoxy, C₁₋₂₀-Alkylthio, C₆₋₂₀-Aryl, C₆₋₂₀-Aryloxy, C₆₋₂₀-Arylthio, C₇₋₂₀-Aralkoxy, C₇₋₂₀-Alkaryloxy, C₇₋₂₀-Alkarylthio, C₇₋₂₀-Aralkyl, C₇₋₂₀-Aralkylthio, Cyano, Carboxylat, C₁₋₂₀-Hydrocarbylcarbonyloxy, C₁₋₂₀-Hydrocarbylsulfinyl, C₃₋₂₀-Hydrocarbylsulfonyl, Amino oder C₁₋₂₀-Dialkylamino ist,
R⁹ unabhängig voneinander bei jedem Auftreten Cyano, Carboxylat, C₁₋₂₀-Hydrocarbylcarbonyloxy, Nitro, Halogen, C₁₋₂₀-Hydrocarbylsulfonyl, C₁₋₂₀-Hydrocarbylsulfinyl, C₁₋₂₀-Alkyl, Amido oder C₁₋₂₀-Hydrocarbyloxy ist,
c unabhängig voneinander bei jedem Auftreten 0 oder eine ganze Zahl von 1 bis 3 ist und
e unabhängig voneinander bei jedem Auftreten 0 oder 1 ist.

11. Verbindung nach einem der Ansprüche 1 bis 10, die ein Oligomer oder Polymer gemäß der Formel IX 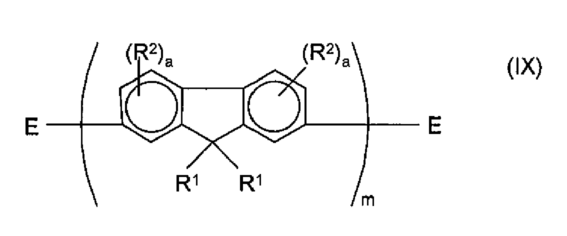 ist, worin:
R¹, R², und a wie in Anspruch 1 definiert sind,
E wie in einem der Ansprüche 4 bis 9 definiert ist und
m für Verbindungen nach Anspruch 2 größer als 1 und ansonsten größer als 10 ist.

12. Verbindung nach einem der Ansprüche 1 bis 10, die ein Oligomer oder Polymer gemäß der Formel X 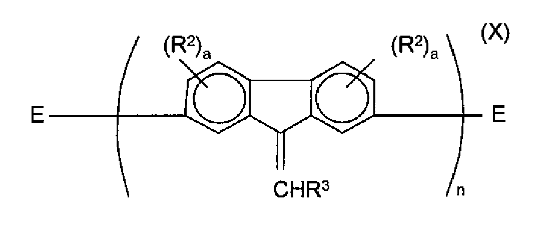 ist, worin:
R², R³, und a wie in Anspruch 1 definiert sind,
E wie in einem der Ansprüche 2 und 6 bis 10 definiert ist und
n für Verbindungen nach Anspruch 2 größer als 1 und ansonsten größer als 10 ist.

13. Verbindung nach Anspruch 12, die ein Oligomer oder Polymer gemäß der Formel XI 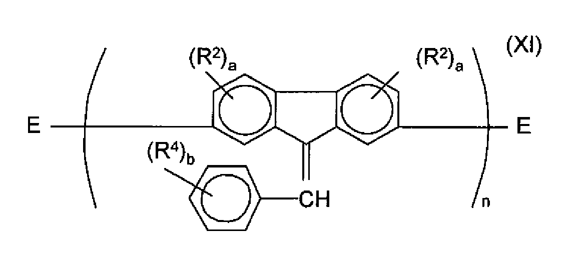 ist, worin:
R² und a wie in Anspruch 1 definiert sind,
R⁴ unabhängig voneinander bei jedem Auftreten Di(C₁₋₂₀-alkyl)amino, C₁₋₂₀-Hydrocarbyloxy, Tri(C₁₋₁₀-alkyl)siloxy oder C₁₋₂₀-Hydrocarbyl ist,
E wie in einem der Ansprüche 2 und 6 bis 10 definiert ist,
b unabhängig voneinander 0 oder eine ganze Zahl von 1 bis 3 ist und
n für Verbindungen nach Anspruch 2 größer als 1 und ansonsten größer als 10 ist.

14. Verbindung nach einem der Ansprüche 1 bis 10, die ein Oligomer oder Polymer gemäß der Formel V 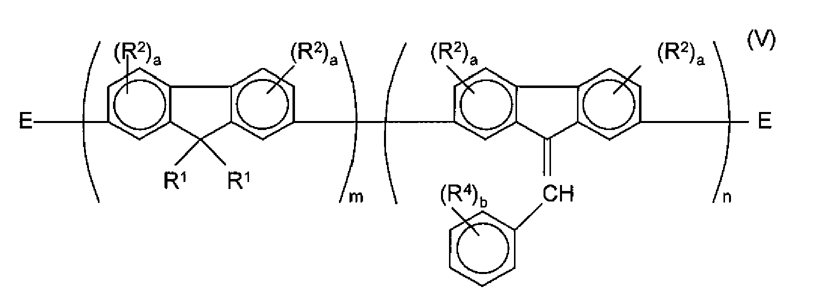 ist, worin:
R¹, R², und a wie in Anspruch 1 definiert sind,
E wie in einem der Ansprüche 2 und 6 bis 10 definiert ist,
R⁴ unabhängig voneinander bei jedem Auftreten Di(C₁₋₂₀-alkyl)amino, C₁₋₂₀-Hydrocarbyloxy, Tri(C₁₋₁₀-alkyl)siloxy oder C₁₋₂₀-Hydrocarbyl ist,
m und n jeweils mindestens 1 sind und
b unabhängig voneinander bei jedem Auftreten 0 oder eine Zahl von 1 bis 3 ist.

15. Verbindung nach einem der Ansprüche 1 bis 11 und 14, worin R¹ C₁₋₁₂-Alkyl, C₆₋₁₀-Aryl oder alkylsubstituiertes Aryl, C₄₋₁₆-Hydrocarbylcarboxylat oder (C₉₋₁₉-Aryl)triaikytsiloxy ist.

16. Verbindung nach einem der Ansprüche 1 bis 11 und 14, worin beide R¹ eine geradkettige oder verzweigte C₅₋₂₀-Ringstruktur (vorbehaltlich dessen, dass es, wenn eine direkte oder indirekte Abhängigkeit von Anspruch 1 besteht, ein aliphatischer C₅₋₁₀-Ring ist) oder eine geradkettige oder verzweigte C₄₋₂₀-Ringstruktur mit einem oder mehreren Heteroatomen ausgewählt aus S, N und O bilden.

17. Verbindung nach Anspruch 16, worin beide R¹ einen aliphatischen C₅₋₀-Ring oder einen aliphatischen C₄₋₁₀-Ring mit einem oder mehreren Heteroatomen ausgewählt aus S und O bilden.

18. Verbindung nach Anspruch 17, worin beide R¹ ein C₅₋₁₀-Cycloalkyl oder sauerstofthaltiges C₄₋₁₀Cycloalkyl bilden.

19. Verbindung nach einem der vorstehenden Ansprüche, worin R² C₁₋₁₂-Alkyl, C₆₋₁₀-Aryl oder alkylsubstituiertes Aryl, C₁₋₁₂-Alkoxy, C₁₋₁₂-Alkoxycarbonyl, C₆₋₁₀-Aryloxycarbonyl oder alkylsubstituiertes Aryloxycarbonyl, C₁₋₁₂-Alkylcarbonyloxy, C₆₋₁₀-Arylcarbonyloxy oder alkylsubstituiertes Arylcarbonyloxy, C₆₋₁₀-Aryloxy oder alkylsubstituiertes Aryloxy, Cyano, Thiocyano, C₆₋₁₂-Thloaryl, C₁₋₂₀-Alkylthio oder Hydroxy ist.

20. Substituierte Fluorenverbindung nach Anspruch 19, worin R² C₁₋₄-Alkoxy, Phenoxy, C₁₋₄-Alkyl, Phenyl oder Cyano ist.

21. Verbindung nach einem der Ansprüche 1 bis 12, 19 und 20, worin R³ ein Kohlenwasserstoffrest ist, ausgewählt aus geradkettigen oder verzweigten aliphatischen C₁₋₂₀-Resten, geradkettigen oder verzweigten aliphatischen C₃₋₂₀-Resten mit einem oder mehreren cycloaliphatischen Ringen, C₆₋₂₀-Arylgruppen und C₇₋₂₀-alkylsubstituierten Arylgruppen, wobei der Kohlenwasserstoffrest optional mit einem oder mehreren Substituenten ausgewählt aus Di(C₁₋₂₀-alkyl)amino, C₁₋₂₀-Hydrocarbyloxy, C₁₋₂₀-Hydrocarbyl und Tri(C₁₋₁₀-alkyl)siloxy substituiert ist.

22. Verbindung nach Anspruch 21, worin R³ ein geradkettiger oder verzweigter aliphatischer C₁₋₂₀-Rest, ein geradkettiger oder verzweigter aliphatischer C₃₋₂₀-Rest mit einem oder mehreren cycloaliphatischen Ringen oder eine Phenylgruppe ist und solch eine Gruppe optional mit einer Di(C₁₋₂₀-alkyl)amino-, C₁₋₂₀-Hydrocarbyl-, Tri(C-₁₋₁₀-alkyl)siloxy- oder C₁₋₂₀-Hydrocarbyloxyeinheit substituiert sein kann.

23. Verbindung nach Anspruch 22, worin R³ ein aliphatischer C₃₋₁₀-Rest, ein aliphatischer C₃₋₁₀-Rest mit einer oder mehreren cycloaliphatischen Einheiten, Phenyl oder Phenyl ist, das mit Di(C₁₋₁₂-alkyl)amino, C₁₋₁₀-Alkoxy, C₆₋₁₀-Aryloxy oder alkylsubstituiertem Aryloxy, C₁₋₁₀-Alkyl oder C₆₋₁₀-Aryl oder alkylsubstituiertem Aryl oder Tri(C₁₋₄-alkyl)siloxy substituiert ist.

24. Verbindung nach Anspruch 23, worin R³ Phenyl oder Phenyl ist, das mit Di(C₁₋₆-alkyl)amino, C₁₋₁₀-Alkoxy oder C₁₋₁₀-Alkyl substituiert ist.

25. Verbindung nach einem der Ansprüche 13 bis 20, worin R⁴ Di(C₁₋₁₂-alkyl)amino, C₁₋₁₀-Alkoxy, C₆₋₁₀-Aryloxy oder alkylsubstituiertes Aryloxy, Tri(C₁₋₄-alkyl)siloxy, C₁₋₁₀-Alkyl, oder C₆₋₁₀-Aryl oder alkylsubstituiertes Aryl ist.

26. Verbindung nach Anspruch 25, worin R⁴ Di(C₁₋₆-alkyl)amino, C₁₋₁₀-Alkoxy oder C₁₋₁₀-Alkyl ist.

27. Verbindung nach Anspruch 25, worin R⁴ C₁₋₁₀-Alkyl, C₆₋₁₀-Aryl oder C₇₋₁₀-Alkylaryl ist.

28. Verbindung nach einem der Ansprüche 13 bis 20 und 25 bis 27, worin b gleich 1 ist.

29. Verbindung nach einem der Ansprüche 1 bis 28, die ein gewichtsmittleres Molekulargewicht von 10.000 oder größer und Polydispersitäten von 3,0 oder weniger aufweist.

30. Substituierte Fluorenverbindung nach einem der Ansprüche 1 bis 29, worin a gleich 0 ist.

31. Verfahren zur Herstellung eines 9-substituierten Fluorenoligomers oder -polymers wie in Anspruch 1 oder Anspruch 2 definiert, umfassend In-Berührung-Bringen eines oder mehrerer 2,7-dihalogen-9-substituierter Fluorene mit einer halogenaromatischen Verbindung in Gegenwart eines Katalysators zur dehalogenierenden Kupplung von Arylhalogeniden.

32. Verfahren zur Herstellung eines 9-substituierten Fluorenoligomers oder -polymers, umfassend In-Berührung-Bringen eines oder mehrerer 2,7-dihalogen-9-substituierter Fluorene und optional einer oder mehrerer halogenaromatischer Verbindungen in Gegenwart einer katalytischen Menge eines divalenten Nickelsalzes, einer mindestens stöchiometrischen Menge von Zinkpulver, eines Trihydrocarbylphosphins in einem polaren Lösungsmittel und einem optionalen Colösungsmittel, das einen aromatischen Kohlenwasserstoff oder Ether enthält, unter Reaktionsbedingungen, die ausreichen, um das entsprechende 9-substituierte Fluorenoligomer oder - polymer auszubilden.

33. Verfahren nach Anspruch 32, worin das 2,7-dihalogen-9-substituierte Fluoren der folgenden Formel entspricht 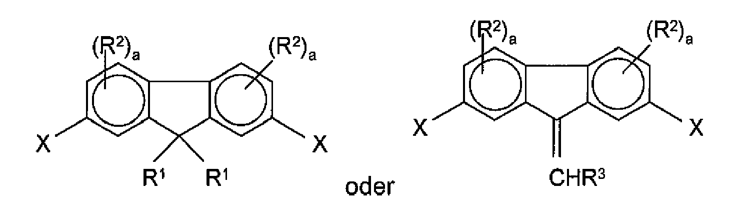 worin:
R¹ unabhängig voneinander bei jedem Auftreten C₁₋₂₀-Hydrocarbyl oder C₁₋₂₀-Hydrocarbyl mit einem oder mehreren Heteroatomen ausgewählt aus S, N, O, P und Si; C₄₋₁₆-Hydrocarbylcarbonyloxy oder (C₉₋₁₆-Aryl)trialkylsiloxy ist oder beide R¹ mit dem 9-Kohlenstoff des Fluorenrings eine C₅₋₂₀-Ringstruktur oder eine C₄₋₂₀-Ringstruktur bilden können, die ein oder mehrere Heteroatome ausgewählt aus S, N und O enthält, R² unabhängig voneinander bei jedem AuftretenC₁₋₂₀-Hydrocarbyl, C₁₋₂₀-Hydrocarbyloxy, C₁₋₂₀-Thioether, C₁₋₂₀-Hydrocarbyloxycarbonyl, C₁₋₂₀-Hydrocarbylcarbonyloxy, Cyano, Thiocyano, C₆₋₁₂-Thioaryl, C₁₋₂₀-Alkylthio oder Hydroxy ist,
R³ unabhängig voneinander bei jedem Auftreten C₁₋₂₀-Hydrocarbyl oder C₁₋₂₀-Hydrocarbyl, substituiert mit Di(C₁₋₂₀-alkyl)amino,
C₁₋₂₀-Hydrocarbyloxy, Tri(C₁₋₁₀-alkyl)siloxy oderC₁₋₂₀-Hydrocarbyl ist,
a unabhängig voneinander bei jedem Auftreten 0 oder 1 ist und
m und n 0 oder nicht-negative Zahlen sind und die Summe von n und m mindestens 1 ist und
X eine Halogengruppe ist.

34. Verfahren nach Anspruch 33, worin das 2,7-dihalogen-9-substituierte Fluoren der Formel I entspricht: 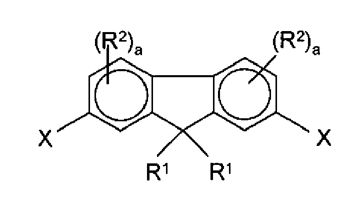 worin:
R¹, R² und a wie in Anspruch 33 definiert sind und
X eine Halogeneinheit ist.

35. Verfahren nach Anspruch 33, worin R¹ C₁₋₁₂-Alkyl, C₆₋₁₀-Aryl oder alkylsubstituiertes Aryl, C₄₋₁₆-Hydrocarbylcarboxylat oder (C₉₋₁₆-Aryl)trialkylsiloxy ist.

36. Verfahren nach Anspruch 35, worin beide R¹ eine geradkettige oder verzweigte C₅₋₂₀-Ringstruktur oder eine geradkettige oder verzweigte C₄₋₂₀-Ringstruktur mit einem oder mehreren Heteroatomen ausgewählt aus S, N und O bilden.

37. Verfahren nach Anspruch 36, worin beide R¹ einen aliphatischen C₅₋₁₀-Ring oder einen aliphatischen C₄₋₁₀Ring mit einem oder mehreren Heteroatomen ausgewählt aus S und O bildern.

38. Verfahren nach Anspruch 37, worin beide R¹ ein C₅₋₁₀-Cycloalkyl oder sauerstoffhaltiges C₄₋₁₀-Cycloalkyl bilden.

39. Verfahren nach Anspruch einem der Ansprüche 33 bis 38, worin R² C₁₋₁₂-Alkyl, C₆₋₁₀-Aryl oder alkylsubstituiertes Aryl, C₁₋₁₂-Alkoxy, C₁₋₁₂-Alkoxycarbonyl, C₆₋₁₀-Aryloxycarbonyl oder alkylsubstituiertes Aryloxycarbonyl, C₁₋₁₂-Alkylcarbonyloxy, C₆₋₁₀-Arylcarbonyloxy oder alkylsubstituiertes Arylcarbonyloxy, C₆₋₁₀-Aryloxy oder alkylsubstituiertes Aryloxy, Cyano, Thiocyano, C₆₋₁₂-Thioaryl, C₁₋₂₀-Alkylthlo oder Hydroxy ist.

40. Verfahren nach Anspruch 39, worin R² C₁₋₄-Alkoxy, Phenoxy, C₁₋₄-Alkyl, Phenyl oder Cyano ist.

41. Verfahren nach Ansprch 33, worin R³ ein Kohlenwasserstoffrest ist, ausgewählt aus geradkettigen oder verzweigten aliphatischen C₁₋₂₀-Resten, geradkettigen oder verzweigten aliphatischen C₃₋₂₀-Resten mit einem oder mehreren cycloaliphatischen Ringen, C₆₋₂₀-Arylgruppen und C₇₋₂₀-alkyl-substituierten Arylgruppen, wobei der Kohlenwasserstoffrest optional mit einem oder mehreren Substituenten ausgewählt aus Di(C₁₋₂₀-alkyl)amino, C₁₋₂₀-Hydrocarbyloxy, C₁₋₂₀-Hydrocarbyl und Tri(C₁₋₁₀-alkyl)siloxy substituiert ist.

42. Verfahren nach Anspruch 41 , worin R³ ein geradkettiger oder verzweigter aliphatischer C₁₋₂₀-Rest, ein geradkettiger oder verzweigter aliphatischer C₃₋₂₀-Rest mit einem oder mehreren cycloaliphatischen Ringen oder eine Phenylgruppe ist und solch eine Gruppe optional mit einer Di(C₁-₂₀-alkyl)amino-, C₁₋₂₀-Hydrocarbyl-, Tri(C₁₋₁₀-alkyl)siloxy- oderC₁₋₂₀-Hydrocarbyloxyeinheit substituiert sein kann.

43. Verfahren nach Anspruch 42, worin R³ ein aliphatischer C₃₋₁₀-Rest, ein aliphatischer C₃₋₁₀-Rest mit einer oder mehreren cycloaliphatischen Einheiten, Phenyl oder Phenyl ist, das mit Di(C₁₋₁₂-alkyl)amino, C₁₋₁₀-Alkoxy, C₆₋₁₀-Aryloxy oder alkylsubstituiertem Aryloxy, C₁₋₁₀-Alkyl oder C₆₋₁₀-Aryl oder alkylsubstituiertem Aryl oder Tri(C₁₋₄-alkyl)siloxy substituiert ist.

44. Verfahren nach Anspruch 43, worin R³ Phenyl oder Phenyl ist, das mit Di(C₁₋₆-alkyl)amino, C₁₋₁₀-Alkoxy oder C₁₋₁₀-Alkyl substituiert ist.

45. Verfahren nach Anspruch 33, worin das 2,7-dihalogen-9-substituierte Fluoren der Formel III entspricht: 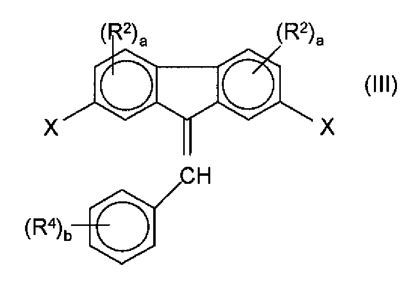 worin:
R² und a wie in Anspruch 33 definiert sind,
R⁴ Di(C₁₋₁₂-alkyl)amino, C₁₋₁₀-Alkoxy, C₆₋₁₀-Aryloxy oder alkylsubstituiertes Aryloxy, Tri(C₁₋₄-alkyl)siloxy, C₁₋₁₀-Alkyl oder C₆₋₁₀-Aryl oder alkylsubstituiertes Aryl ist und
b gleich 0 oder eine Zahl von 1 bis 3 ist.

46. Verfahren nach Anspruch 45, worin R⁴ Di(C₁₋₆-a)kyl)amino, C₁₋₁₀-Alkoxy oder C₁₋₁₀-Alkyl ist.

47. Verfahren nach Anspruch 45 oder Anspruch 46, worin b gleich 1 ist.

48. Verfahren nach einem der Ansprüche 31 bis 47, worin die halogenaromatische Verbindung die Formel X-Ar aufweist, worin X ein Halogen ist und Ar unabhängig voneinander bei jedem Auftreten ein Aryl oder ein Aryl ist, substituiert mit einer Gruppe, die zur Vernetzung oder Kettenverlängerung fähig ist, oder eine Trialkylsiloxyeinheit ist.

49. Verfahren nach Anspruch 48, worin Ar Aryl oder Aryl ist, das mit einer Hydroxy-, einer Glycidylether-, Acrylatester-, Methacrylatester-, Ethenyl-, Ethinyl-, Maleimid-, Nadimid- oder Trifluorvinylethereinheit oder einer Benzocyclobutengruppe substituiert ist.

50. Verfahren nach Anspruch 49, worin Ar eine Arylgruppe ist, die mit einem Acrylat- oder Methacrylatester entsprechend der Formel 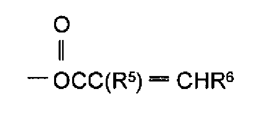 oder einer Ethenylgruppe entsprechend der Formel
- C(R⁷) = C(R⁷)H
substituiert ist,
worin:
R⁵ unabhängig voneinander bei jedem Auftreten Wasserstoff oder C₁₋₄-Alkyl ist,
R⁶ unabhängig voneinander bei jedem Auftreten Wasserstoff, C₁₋₂₀-Hydrocarbyl oder C₁₋₂₀-Hydrocarbyloxy ist,
R⁷ unabhängig voneinander bei jedem Auftreten Wasserstoff, C₁₋₂₀-Hydrocarbyl oder C₁₋₂₀-Hydrocarbyloxy ist,

51. Verfahren nach Anspruch 50, worin Ar eine Arylgruppe ist, die mit einem Benzocyclobuten entsprechend der Formel, 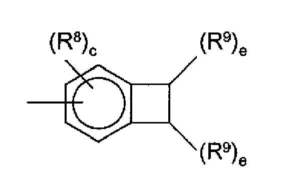 substituiert ist,
worin
R⁸ unabhängig voneinander bei jedem Auftreten C₁₋₂₀-Alkyl, C₁₋₂₀-Alkoxy, C₁₋₂₀-Alkylthlo, C₆₋₂₀-Aryl, C₆₋₂₀-Aryloxy, C₆₋₂₀-Arylthio, C₇₋₂₀-Aralkoxy, C₇₋₂₀-Alkaryloxy, C₇₋₂₀-Alkarylthio, C₇₋₂₀-Aralkyl, C₇₋₂₀-Aralkylthlo, Cyano, Carboxylat, C₁₋₂₀-Hydrocarbylcarbonyloxy, C₁₋₂₀-Hydrocarbylsulfonyl, C₁₋₂₀-Hydrocarbylsulfinyl, Amino oder C₁₋₂₀-Dialkylamino ist,
R⁹ unabhängig voneinander bei jedem Auftreten Cyano, Carboxylat, C₁₋₂₀-Hydrocarbylcarbonyloxy, Nitro, Halogen, C₁₋₂₀-Hydrocarbylsulfonyl, Hydrocarbylsulfinyl, C₁₋₂₀-Alkyl, Amido oder C₁₋₂₀-Hydrocarbyloxy ist,
b unabhängig voneinander bei jedem Auftreten 0 oder eine ganze Zahl von 1 bis 3 ist,
c unabhängig voneinander bei jedem Auftreten 0 oder eine ganze Zahl von 1 bis 3 ist und
e unabhängig voneinander bei jedem Auftreten 0 oder 1 ist.

52. Verfahren nach einem der Ansprüche 31 bis 51, worin das Molverhältnis von 2,7-dihalogen-9-substituiertem Fluoren zu halogenaromatischer Verbindung von 0,5:1 bis 50:1 reicht.

53. Verfahren nach Anspruch 52, worin das Verfahren in Gegenwart von einem divalenten Nickelsalze, das in einer Menge von 0,01 to 20 Mol-%, bezogen auf die Menge von halogenaromatische Verbindung und
2,7-Dihalogenfluoren, vorliegt, Zinkpulver, das in einer Menge von 100 Mol-% bis 300 Mol-%, bezogen auf halogenaromatische Verbindung und 2,7-Dihalogenfluoren, vorliegt, Triarylphosphin, das in einer Menge von 10 bis 50 Mol-%, bezogen auf halogenaromatische Verbindung und 2,7- Dihalogenfluoren, und einer Verbindung, die fähig ist, die Reaktion zu beschleunigen und in einer Menge von 100 bis 150 Mol-%, bezogen auf das divalente Salz, durchgeführt wird.

54. Folie aus einem Oligomer oder Polymer wie in einem der Ansprüche 1 bis 30 definiert.

55. Beschichtung aus einem Polymer wie in einem der Ansprüche 1 bis 30 definiert.

56. Polymere lichtaussendende Diode, die eine Folie oder eine Beschichtung aufweist, wie in Anspruch 54 oder 55 definiert.

## Revendications

1. Composé de formule : 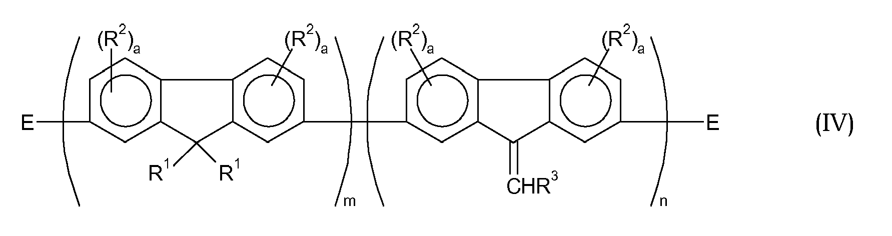 dans laquelle:
chaque E est indépendamment un halogène, un radical aryle ou aryle substitué par un groupe réactif pouvant subir une extension de chaîne ou une réticulation ou un groupe trialkylsiloxy ;
chaque R¹ est indépendamment un radical hydrocarbyle en C₁ à C₂₀ ou hydrocarbyle en C₁ à C₂₀ contenant un ou plusieurs hétéroatomes choisis parmi S, N, O, P et Si ; hydrocarbylcarbonyloxy en C₄ à C₁₆, ou (aryl en C₉ à C₁₆)trialkylsiloxy, ou bien deux R¹, ensemble avec l'atome de carbone 9 du cycle fluorène, peuvent former une structure cyclique aliphatique en C₅ à C₁₀ ou une structure cyclique en C₄ à C₂₀ contenant un ou plusieurs hétéroatomes choisis parmi S, N et O;
chaque R² est indépendamment un radical hydrocarbyle en C₁ à C₂₀, hydrocarbyloxy en C₁ à C₂₀, thioéther en C₁ à C₂₀, hydrocarbyloxycarbonyle en C₁ à C₂₀, hydrocarbyl-carbonyloxy en C₁ à C₂₀, cyano, thiocyano, thioaryle en C₆ à C₁₂, alkylthio en C₁ à C₂₀ ou hydroxy ;
chaque R³ est indépendamment un radical hydrocarbyle en C₁ à C₂₀ ou hydrocarbyle en C₁ à C₂₀ substitué par un radical di(alkyl en C_{1.} à C₂₀)amino, hydrocarbyloxy en C₁ à C₂₀, tri(alkyl en C₁ à C₁₀)siloxy ou hydrocarbyle en C₁ à C₂₀ ;
chaque a vaut indépendamment 0 ou 1 ; et
m et n valent 0 ou sont des entiers non négatifs, et la somme de n et m est au moins égale à 10 et,
dans lequel les polymères ont une polydispersité inférieure à 5.

2. Composé de formule : 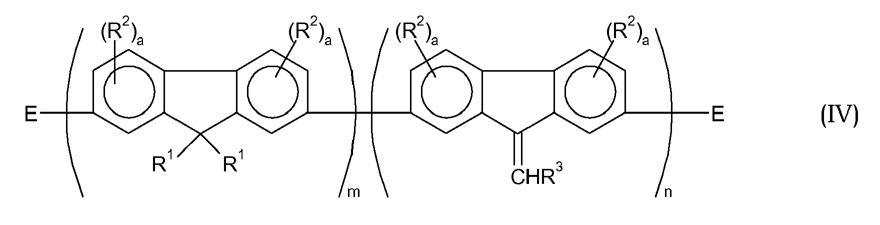 dans laquelle:
E est un radical phénolique, phényle substitué par du cyanato ou benzocyclobutène,
chaque R¹ est indépendamment un radical hydrocarbyle en C₁ à C₂₀ ou hydrocarbyle en C₁ à C₂₀ contenant un ou plusieurs hétéroatomes choisis parmi S, N, O, P et Si ; hydrocarbylcarbonyloxy en C₄ à C₁₆, ou (aryl en C₉ à C₁₆)trialkylsiloxy, ou bien deux R¹, ensemble avec l'atome de carbone 9 du cycle fluorène, peuvent former une structure cyclique aliphatique en C₅ à C₂₀ ou une structure cyclique en C₄ à C₂₀ contenant un ou plusieurs hétéroatomes choisis parmi S, N et O;
chaque R² est indépendamment un radical hydrocarbyle en C₁ à C₂₀, hydrocarbyloxy en C₁ à C₂₀, thioéther en C₁ à C₂₀, hydrocarbyloxycarbonyle en C₁ à C₂₀, hydrocarbyl-carbonyloxy en C₁ à C₂₀, cyano, thiocyano, thioaryle en C₆ à C_{12,} alkylthio en C₁ à C₂₀ ou hydroxy ;
chaque R³ est indépendamment un radical hydrocarbyle en C₁ à C₂₀ ou hydrocarbyle en C₁ à C₂₀ substitué par un radical di(alkyl en C₁ à C₂o)amino, hydrocarbyloxy en C₁ à C₂₀, tri(alkyl en C₁ à C₁₀)siloxy ou hydrocarbyle en C₁ à C₂₀ ;
chaque a vaut indépendamment O ou 1 ; et
m et n valent O ou sont des entiers non négatifs, et la somme de n et m est supérieur à 1 et,
dans lequel les polymères ont une polydispersité inférieure à 5.

3. Composé selon la revendication 2, dans lequel la somme de n et m est au moins 10.

4. Composé selon la revendication 1 ou la revendication 3, dans lequel m est un nombre de 1 ou plus et n vaut O ou un nombre de 1 ou plus (limitation de n et m selon la revendication de référence).

5. Composé selon l'une des revendications 2 à 4, dans lequel m est un nombre de 1 à 100 et n vaut O ou un nombre de 1 à 100 (limitation de n et m selon la revendication de référence).

6. Composé selon l'une des revendications 1, 4 et 5, dans lequel chaque E est indépendamment un radical aryle ; aryle substitué par un groupement hydroxy, glycidyléther, acrylate (ester), méthacrylate (ester), éthényle, éthynyle, maléimide, nadimide, trialkylsiloxy, trifluorovinyléther ; ou benzocyclobutène.

7. Composé selon la revendication 1, dans lequel E est un halogène ou un radical cyanato, aryle ou aryle substitué par un groupe réactif pouvant subir une extension de chaîne ou une réticulation ou un groupement trialkylsiloxy.

8. Composé selon la revendication 7, dans lequel E est un radical aryle ou aryle substitué par un groupe réactif pouvant subir une extension de chaîne ou une réticulation ou un radical trialkylsiloxy.

9. Composé selon la revendication 8, dans lequel E est un groupement aryle ayant un groupe réactif acrylate ou méthacrylate (ester) correspondant à la formule : 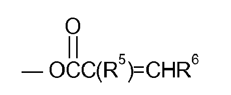 ou un groupement éthényle correspondant à la formule :
-C(R⁷)=C(R⁷)H
dans lesquelles :
chaque R⁵ est indépendamment l'hydrogène ou un radical alkyle en C₁ à C₄;
chaque R⁶ est indépendamment l'hydrogène ou un radical hydrocarbyle en C₁ à C₂₀ ou hydrocarbyloxy en C₁ à C₂₀ ; et
chaque R⁷ est indépendamment l'hydrogène ou un radical hydrocarbyle en C₁ à C₂₀ ou hydrocarbyloxy en C₁ à C₂₀.

10. Composé selon la revendication 2, dans lequel E est un groupement benzocyclobutène correspondant à la formule : 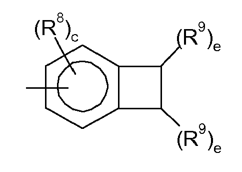 dans laquelle:
chaque R⁸ est indépendamment un radical alkyle en C₁ à C₂₀, alcoxy en C₁ à C₂₀, alkylthio en C₁ à C₂₀, aryle en C₆ à C₂₀, aryloxy en C₆ à C₂₀, arylthio en C₆ à C₂₀, aralcoxy en C₇ à C₂₀, alcaryloxy en C₇ à C₂₀, alcarylthio en C₇ à C₂₀, aralkyle en C₇ à C₂₀, aralkylthio en C₇ à C₂₀, cyano, carboxylate, hydrocarbylcarbonyloxy en C₁ à C₂₀, hydrocarbylsulfinyle en C₁ à C₂₀, hydrocarbylsulfonyle en C₁ à C₂₀, amino ou dialkylamino en C₁ à C₂₀ ;
chaque R⁹ est indépendamment un radical cyano, carboxylate, hydrocarbylcarbonyloxy en C₁ à C₂₀, nitro, halogéno, hydrocarbylsulfonyle en C₁ à C₂₀, hydrocarbylsulfinyle en C₁ à C₂₀, alkyle en C₁ à C₂₀, amido ou hydrocarbyloxy en C₁ à C₂₀ ;
chaque c est indépendamment 0 ou un entier de 1 à 3 ; et
chaque e est indépendamment 0 ou 1.

11. Composé selon l'une des revendications 1 à 10, qui est un oligomère ou polymère conforme à la formule IX : 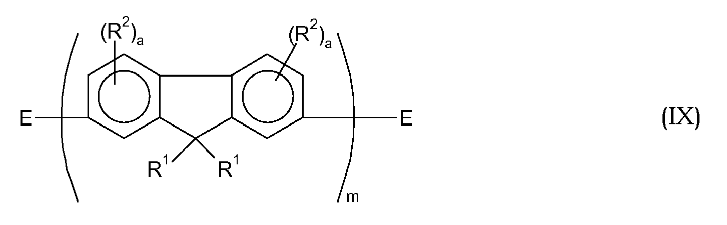 dans laquelle:
R¹, R² et a sont tels que définis dans la revendication 1 ;
E est tel que défini dans l'une quelconque des revendications 4 à 9 ; et
m est supérieur à 1 pour les composés de la revendication 2 et sinon supérieur à 10.

12. Composé selon l'une quelconque des revendications 1 à 10, qui est un oligomère ou polymère conforme à la formule X : 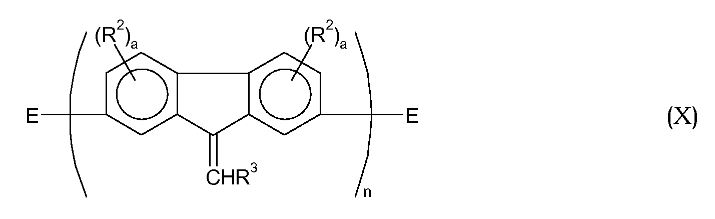 dans laquelle:
R², R³ et a sont tels que définis dans la revendication 1 ;
E est tel que défini dans l'une quelconque des revendications 2 et 6 à 10 ; et
n est supérieur à 1 pour les composés de la revendication 2 et sinon supérieur à 10.

13. Composé selon la revendication 12, qui est un oligomère ou polymère conforme à la formule XI : 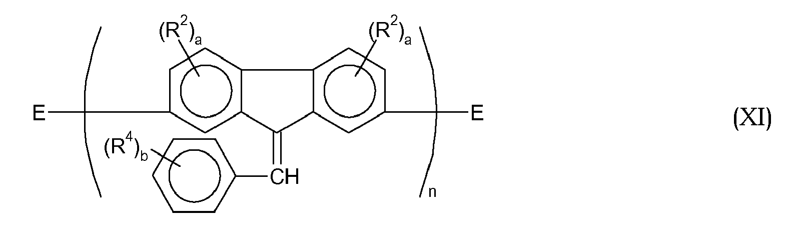 dans laquelle:
R² et a sont tels que définis dans la revendication 1 ;
Chaque R⁴ est indépendamment un radical di(alkyl en C₁ à C₂₀)amino, hydrocarbyloxy en C₁ à C₂₀, tri(alkyl en C₁ à C₁₀)siloxy ou hydrocarbyl en C₁ à C₂₀ ;
E est tel que défini dans l'une quelconque des revendications 2 et 6 à 10;
b est indépendamment 0 ou un nombre de 1 à 3 ; et
n est supérieur à 1 pour les composés de la revendication 2 et sinon supérieur à 10.

14. Composé selon l'une quelconque des revendications 1 à 10, qui est un oligomère ou polymère conforme à la formule V : 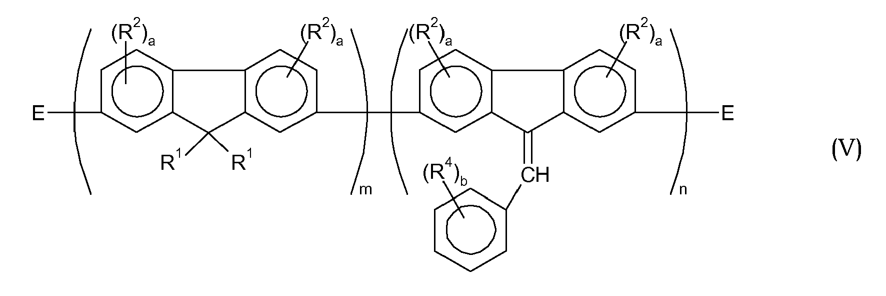 dans laquelle:
R¹, R² et a sont tels que définis dans la revendication 1 ;
E est tel que défini dans l'une quelconque des revendications 2 et 6 à 10;
Chaque R⁴ est indépendamment un radical di(alkyl en C₁ à C₂₀)amino, hydrocarbyloxy en C₁ à C₂₀, tri(alkyl en C₁ à C₁₀)siloxy ou hydrocarbyl en C₁ à C₂₀ ;
m et n valent chacun au moins 1 ; et
chaque b est indépendamment O ou un nombre de 1 à 3.

15. Composé selon l'une quelconque des revendications 1 à 10 et 14, dans lequel R¹ est un radical alkyle en C₁ à C₁₂, aryle ou aryle à substitution alkyle en C₆ à C₁₀, carboxylate hydrocarbyle en C₄ à C₁₆, ou (aryl en C₉ à C₁₆)trialkylsiloxy.

16. Composé selon l'une quelconque des revendications 1 à 11 et 14, dans lequel les deux R¹ forment une structure cyclique à chaîne droite ou ramifiée en C₅ à C₂₀ (l'objet étant une structure aliphatique en C₅à C₁₀ quand il dépend directement ou indirectement de la revendication 1) ou une structure cyclique à chaîne droite ou ramifiée en C₄ à C₂₀ contenant un ou plusieurs hétéroatomes choisis parmi S, N et O.

17. Composé selon la revendication 16, dans lequel les deux R¹ forment un cycle aliphatique en C₅ à C₁₀ ou un cycle aliphatique en C₄ à C₁₀ contenant un ou plusieurs hétéroatomes choisis parmi S et O.

18. Composé selon la revendication 17, dans lequel les deux R¹ forment un radical cycloalkyle en C₅ à C₁₀ ou cycloalkyle en C₄ à C₁₀ contenant de l'oxygène.

19. Composé selon l'une quelconque des revendications précédentes, dans lequel R² est un radical alkyle en C₁ à C₁₂, aryle ou aryle à substitution alkyle en C₆ à C₁₀, alcoxy en C₁ à C₁₂, alcoxycarbonyle en C₁ à C₁₂, aryloxycarbonyle ou aryloxycarbonyle à substitution alkyle en C₆ à C₁₀, alkylcarbonyloxy en C₁ à C₁₂, arylcarbonyloxy ou arylcarbonyloxy à substitution alkyle en C₆ à C₁₀, aryloxy ou aryloxy à substitution alkyle en C₆ à C₁₀, cyano, thiocyano, thioaryle en C₆ à C_{12,} alkylthio en C₁ à C₂₀, ou hydroxy.

20. Composé de fluorène substitué selon la revendication 19, dans lequel R² est un radical alcoxy en C₁ à C₄, phénoxy, alkyle en C₁ à C₄, phényle ou cyano.

21. Composé selon l'une quelconque des revendications 1 à 12, 19, et 20, dans lequel R³ est un radical hydrocarbyle choisi parmi les radicaux aliphatiques à chaîne droite ou ramifiée en C₁ à C₂₀, les radicaux aliphatiques à chaîne droite ou ramifiée en C₃ à C₂₀ contenant un ou plusieurs cycles cycloaliphatiques, les radicaux aryle en C₆ à C₂₀, et les radicaux aryle en C₇ à C₂₀ à substitution alkyle dans lesquels le groupement hydrocarbyle est éventuellement substitué par un ou plusieurs substituants choisis parmi les radicaux di(alkyl en C₁ à C₂₀)amino, hydrocarbyloxy en C₁ à C₂₀, hydrocarbyle en C₁ à C₂₀ et tri(alkyl en C₁ à C₁₀)siloxy.

22. Composé selon la revendication 21, dans lequel R³ est un radical aliphatique à chaîne droite ou ramifiée en C₁ à C₂₀, un radical aliphatique à chaîne droite ou ramifiée en C₃ à C₂₀ contenant un ou plusieurs cycles cycloaliphatiques ou un groupement phényle et ce groupement peut éventuellement être substitué par un groupement di(alkyl en C₁ à C₂₀)amino, hydrocarbyle en C₁ à C₂₀, tri(alkyl en C₁ à C₁₀)siloxy ou hydrocarbyloxy en C₁ à C₂₀.

23. Composé selon la revendication 22, dans lequel R³ est un radical aliphatique en C₃ à C₁₀, un radical aliphatique en C₃ à C₁₀ contenant un ou plusieurs groupements cycloaliphatiques, un radical phényle ou phényle substitué par un radical di(alkyl en C₁ à C₁₀)amino, un radical alcoxy en C₁ à C₁₀, aryloxy ou aryloxy à substitution alkyle en C₆ à C₁₀, alkyle en C₁ à C₁₀ ou aryle ou aryle à substitution alkyle en C₆ à C₁₀ ou tri(alkyl en C₁ à C₄)siloxy.

24. Composé selon la revendication 23, dans lequel R³ est un radical phényle ou phényle substitué par un radical di(alkyl en C₁ à C₆)amino, alcoxy en C₁ à C₁₀ ou alkyle en C₁ à C₁₀.

25. Composé selon l'une quelconque des revendications 13 à 20, dans lequel R⁴ est un radical di(alkyl en C₁ à C₁₂)amino, alcoxy en C₁ à C₁₀, aryloxy ou aryloxy à substitution alkyle en C₆ à C₁₀, tri(alkyl en C₁ à C₄)siloxy, alkyle en C₁ à C₁₀, ou aryle ou aryle à substitution alkyle en C₆ à C₁₀.

26. Composé selon la revendication 25, dans lequel R⁴ est un radical di(alkyl en C à C₆)amino, alcoxy en C₁ à C₁₀ ou alkyle en C₁ à C₁₀.

27. Composé selon la revendication 25, dans lequel R⁴ est un radical alkyle en C₁ à C₁₀, aryle en C₆ à C₁₀ ou alkylaryle en C₇ à C₁₀.

28. Composé selon l'une quelconque des revendications 13 à 20, et 25 à 27, dans lequel b vaut 1.

29. Composé selon l'une quelconque des revendications 1 à 28, qui a un poids moléculaire moyen en poids de 10 000 ou plus et une polydispersité de 3,0 ou moins.

30. Composé de fluorène substitué selon l'une quelconque des revendications 1 à 29, dans lequel a vaut O.

31. Procédé pour préparer un oligomère ou polymère de fluorène 9-substitué, qui comprend la mise en contact d'un ou plusieurs 2,7-dihalogénofluorènes 9-substitués avec un composé halogénoaromatique en présence d'un catalyseur pour le couplage par déshalogénation d'halogénures d'aryle.

32. Procédé pour préparer un oligomère ou polymère de fluorène 9-substitué, qui comprend la mise en contact d'un ou plusieurs 2,7-dihalogénofluorènes 9-substitués et éventuellement d'un ou plusieurs composés halogéno-aromatiques, en présence d'une quantité catalytique d'un sel de nickel divalent, d'au moins une quantité stoechiométrique de poudre de zinc, d'une trihydrocarbylphosphine dans un solvant polaire et d'un co-solvant facultatif comprenant un hydrocarbure aromatique ou de l'éther, dans des conditions réactionnelles suffisantes pour former l'oligomère ou polymère de fluorène 9-substitué correspondant.

33. Procédé selon la revendication 32, dans lequel le 2,7-dihalogénofluorène 9-substitué correspond à la formule suivante : 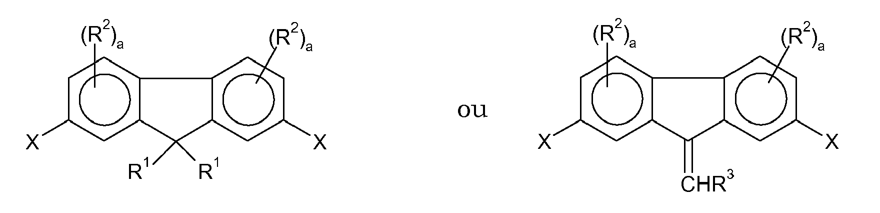 dans laquelle:
chaque R¹ est indépendamment un radical hydrocarbyle en C₁ à C₂₀ ou hydrocarbyle en C₁ à C₂₀ contenant un ou plusieurs hétéroatomes choisis parmi S, N, O, P et Si ; hydrocarbylcarbonyloxy en C₄ à C₁₆, ou (aryl en C₉ à C₁₆)trialkylsiloxy, ou bien deux R¹, ensemble avec l'atome de carbone 9 du cycle fluorène, peuvent former une structure cyclique aliphatique en C₅ à C₂₀ ou une structure cyclique en C₄ à C₂₀ contenant un ou plusieurs hétéroatomes choisis parmi S, N et O;
chaque R² est indépendamment un radical hydrocarbyle en C₁ à C₂₀, hydrocarbyloxy en C₁ à C₂₀, thioéther en C₁ à C₂₀, hydrocarbyloxycarbonyle en C₁ à C₂₀, hydrocarbyl-carbonyloxy en C₁ à C₂₀, cyano, thiocyano, thioaryle en C₆ à C₁₂, alkylthio en C₁ à C₂₀ ou hydroxy ;
chaque R³ est indépendamment un radical hydrocarbyle en C₁ à C₂₀ ou hydrocarbyle en C₁ à C₂₀ substitué par un radical di(alkyl en C₁ à C₂₀)amino, hydrocarbyloxy en C₁ à C₂₀, tri(alkyl en C₁ à C₁₀)siloxy ou hydrocarbyle en C₁ à C₂₀ ;
chaque a vaut indépendamment 0 ou 1 ; et
m et n valent 0 ou sont des entiers non négatifs, et la somme de n et m est au moins égale à 1 et,
X est un groupement halogène.

34. Procédé selon la revendication 33, dans lequel le 2,7-dihalogénofluorène 9-substitué correspond à la formule 1 : 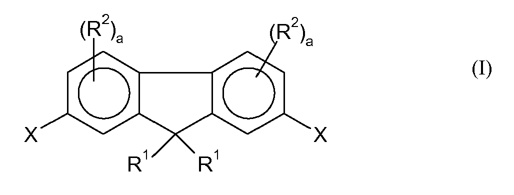 où
R¹, R², et a sont tels que définis à la revendication 33 et
X est un groupement halogène.

35. Procédé selon la revendication 33, dans lequel R¹ est un alkyl en C₁ à C₁₂, un aryle ou aryle à substitution alkyl en C₆ à C₁₀, carboxylate hydrocarbyle en C₄ à C₁₆ ou (aryle en C₉ à C₁₆)trialkylsiloxy.

36. Procédé selon la revendication 35, dans lequel les R1 ensemble une structure cyclique à chaîne droite ou ramifiée en C5 à C20 ou structure cyclique à chaîne droite ou ramifiée en C4 à c20 contenant un ou plusieurs hétéroatomes choisis parmi S, N et O.

37. Procédé selon la revendication 36, dans lequel les deux R¹ forment un cycle aliphatique en C₅ à C₁₀ ou un cycle aliphatique en C₄ à C₁₀ contenant un ou plusieurs hétéroatomes choisis parmi S et O.

38. Procédé selon la revendication 37, dans lequel les deux R¹ forment un radical cycloalkyle en C₅ à C₁₀ ou cycloalkyle en C₄ à C₁₀ contenant de l'oxygène.

39. Procédé selon l'une des revendications 33 à 38, dans lequel R² est un radical alkyle en C₁ à C₁₂, aryle ou aryle à substitution alkyle en C₆ à C₁₀, alcoxy en C₁ à C₁₂, alcoxycarbonyle en C₁ à C₁₂, aryloxycarbonyle ou aryloxycarbonyle à substitution alkyle en C₆ à C₁₀, alkylcarbonyloxy en C₁ à C₁₂, arylcarbonyloxy ou arylcarbonyloxy à substitution alkyle en C₆ à C₁₀, aryloxy ou aryloxy à substitution alkyle en C₆ à C₁₀, cyano, thiocyano, thioaryle en C₆ à C₁₂, alkylthio en C₁ à C₂₀, ou hydroxy.

40. Procédé selon la revendication 39, dans lequel R² est un radical alcoxy en C₁ à C₄, phénoxy, alkyle en C₁ à C₄, phényle ou cyano.

41. Procédé selon la revendication 33, dans lequel R³ est un radical hydrocarbyle choisi parmi les radicaux aliphatiques à chaîne droite ou ramifiée en C₁ à C₂₀, les radicaux aliphatiques à chaîne droite ou ramifiée en C₃ à C₂₀ contenant un ou plusieurs cycles cycloaliphatiques, les radicaux aryle en C₆ à C₂₀, et les radicaux aryle en C₇ à C₂₀ à substitution alkyle dans lesquels le groupement hydrocarbyle est éventuellement substitué par un ou plusieurs substituants choisis parmi les radicaux di(alkyl en C₁ à C₂₀)amino, hydrocarbyloxy en C₁ à C₂₀, hydrocarbyle en C₁ à C₂₀ et tri(alkyl en C₁ à C₁₀)siloxy.

42. Procédé selon la revendication 41, dans lequel R³ est un radical aliphatique à chaîne droite ou ramifiée en C₁ à C₂₀, un radical aliphatique à chaîne droite ou ramifiée en C₃ à C₂₀ contenant un ou plusieurs cycles cycloaliphatiques ou un groupement phényle et ce groupement peut éventuellement être substitué par un groupement di(alkyl en C₁ à C₂₀)amino, hydrocarbyle en C₁ à C₂₀, tri(alkyl en C₁ à C₁₀)siloxy ou hydrocarbyloxy en C₁ à C₂₀.

43. Procédé selon la revendication 42, dans lequel R³ est un radical aliphatique en C₃ à C₁₀, un radical aliphatique en C₃ à C₁₀ contenant un ou plusieurs groupements cycloaliphatiques, un radical phényle ou phényle substitué par un radical di(alkyl en C₁ à C₁₀)amino, un radical alcoxy en C₁ à C₁₀, aryloxy ou aryloxy à substitution alkyle en C₆ à C₁₀, alkyle en C₁ à C₁₀ ou aryle ou aryle à substitution alkyle en C₆ à C₁₀ ou tri(alkyl en C₁ à C₄)siloxy.

44. Procédé selon la revendication 43, dans lequel R³ est un radical phényle ou phényle substitué par un radical di(alkyl en C₁ à C₆)amino, alcoxy en C₁ à C₁₀ ou alkyle en C₁ à C₁₀.

45. Procédé selon la revendication 33, dans lequel le 2,7-dihalogénofluorène 9-substitué coorespond à la formule III, 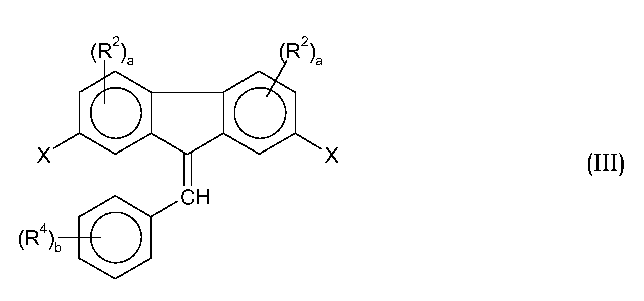 dans laquelle,
R2 est tel que défini dans la revendication 33 ;
R4 est un radical di(alkyl en C₁ à C₁₂)amino, alcoxy en C₁ à C₁₀, aryloxy ou aryloxy à substitution alkyle en C₆ à C₁₀, tri(alkyl en C₁ à C₄)siloxy, alkyle en C₁ à C₁₀, ou aryle ou aryle à substitution alkyle en C₆ à C₁₀ et
B vaut 0 ou un nombre de 1 à 3.

46. Procédé selon la revendication 45, dans lequel R⁴ est un radical di(alkyl en C₁ à C₆)amino, alcoxy en C₁ à C₁₀ ou alkyl en C₁ à C₁₀.

47. Procédé selon la revendication 45 ou la revendication 46, dans lequel b vaut 1.

48. Procédé selon l'une quelconque des revendications 31 à 47, dans lequel le composé halogénoaromatique est de formule X-Ar où X est un halogène et chaque Ar est indépendamment un groupement aryle ou aryle substitué par un groupement pouvant subir une réticulation ou une extension de chaîne ou un groupement trialkylsiloxy.

49. Procédé selon la revendication 48, dans lequel Ar est un radical aryle ou aryle substitué par un groupement hydroxy, glycidyléther, ester acrylate, ester méthacrylate, éthényle, éthynyle, maléimide, nadimide ou trifluorovinyléther ou un radical benzocyclobutène.

50. Procédé selon la revendication 49, dans lequel Ar est un groupe aryle substitué par un acrylate ou méthacrylate (ester) correspondant à la formule : 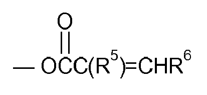 ou un groupement éthényle correspondant à la formule :
-C(R⁷)=C(R⁷)H
dans lesquelles :
chaque R⁵ est indépendamment l'hydrogène ou un radical alkyle en C₁ à C₄ ;
chaque R⁶ est indépendamment l'hydrogène ou un radical hydrocarbyle en C₁ à C₂₀ ou hydrocarbyloxy en C₁ à C₂₀ ;
chaque R⁷ est indépendamment l'hydrogène ou un radical hydrocarbyle en C₁ à C₂₀ ou hydrocarbyloxy en C₁ à C₂₀.

51. Procédé selon la revendication 50, dans lequel Ar est un groupe aryle substitué par un benzocyclobutène correspondant à la formule : 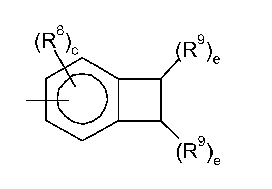 dans laquelle:
chaque R⁸ est indépendamment un radical alkyle en C₁ à C₂₀, alcoxy en C₁ à C₂₀, alkylthio en C₁ à C₂₀, aryle en C₆ à C₂₀, aryloxy en C₆ à C₂₀, arylthio en C₆ à C₂₀, aralcoxy en C₇ à C₂₀, alcaryloxy en C₇ à C₂₀, alcarylthio en C₇ à C₂₀, aralkyle en C₇ à C₂₀, aralkylthio en C₇ à C₂₀, cyano, carboxylate, hydrocarbylcarbonyloxy en C₁ à C₂₀, hydrocarbylsulfonyle en C₁ à C₂₀, hydrocarbylsulfinyle, amino ou dialkylamino en C₁ à C₂₀ ;
chaque R⁹ est indépendamment un radical cyano, carboxylate, hydrocarbylcarbonyloxy en C₁ à C₂₀, nitro, halogéno, hydrocarbylsulfonyle en C₁ à C₂₀, hydrocarbyl-sulfinyle en C₁ à C₂₀, alkyle en C₁ à C₂₀, amido ou hydrocarbyloxy en C₁ à C₂₀ ;
chaque b est indépendamment 0 ou un entier de 1 à 3 ;
chaque c est indépendamment 0 ou un entier de 1 à 3 ; et
chaque e est indépendamment 0 ou 1.

52. Procédé selon l'une quelconque des revendications 31 à 51, dans lequel le rapport molaire du 2,7-dihalogénofluorène 9-substitué au composé halogéno-aromatique est de 0,5/1 à 50/1.

53. Procédé selon la revendication 52, dans lequel le procédé est mis en oeuvre en présence d'un sel de nickel divalent présent en une quantité de 0,01 à 20 % en moles par rapport à la quantité de composé halogénoaromatique et de 2,7-dihalogénofluorène, de poudre de zinc présente en une quantité de 100 à 300 % en moles par rapport au composé halogénoaromatique et au 2,7-dihalogénofluorène, de triarylphosphine présente en une quantité de 10 à 50 % en moles par rapport au composé halogénoaromatique et au 2,7-dihalogénofluorène, et d'un composé pouvant accélérer la réaction présent en une quantité de 10 à 150 % en moles par rapport au sel divalent.

54. Film d'un oligomère ou polymère tel que défini dans l'une quelconque des revendications 1 à 30.

55. Revêtement d'un polymère tel que défini dans l'une quelconque des revendications 1 à 30.

56. Diode polymère à luminescence comprenant un film ou revêtement tel que défini dans la revendication 54 ou la revendication 55.
